# EUROPÄISCHE PATENTSCHRIFT

(11) **EP 3 435 939 B1**
(45) Veröffentlichungstag und Bekanntmachung des Hinweises auf die Patenterteilung: **24.06.2026**
(21) Anmeldenummer: 17714688.3
(22) Anmeldetag: 28.03.2017
(51) Int. Cl.: A61F 13/00, A61F 13/02, A61K 9/70, G09F 3/00

(54) **TRANSDERMALES APPLIKATIONSSYSTEM MIT INFORMATIONSSCHICHT**
TRANSDERMAL APPLICATION SYSTEM WITH INFORMATION LAYER
SYSTEM D'APPLICATION TRANSDERMIQUE AVEC UNE COUCHE D'INFORMATION

(30) Priorität: 31.03.2016 DE 102016105889
(43) Veröffentlichungstag der Anmeldung: 06.02.2019
(73) Patentinhaber: AdhexPharma SAS, 75016 Paris (FR)
(72) Erfinder: GEGENFURTNER, Klaus, 83707 Bad Wiessee (DE); KAFFL, Hubert, 83730 Fischbachau (DE); DR. RODLER, Stefanie, 83626 Valley (DE)
(74) Vertreter: Beckord & Niedlich Patentanwälte PartG mbB
(86) Internationale Anmeldenummer: PCT/EP2017/057274
(87) Internationale Veröffentlichungsnummer: WO 2017/167728

(56) Entgegenhaltungen:
- WO-A1-00/04480
- WO-A1-87/02241
- WO-A1-99/09960
- WO-A2-99/12529
- DE-A1- 19 708 674
- DE-U1- 9 409 784

## Beschreibung

Die vorliegende Erfindung betrifft ein transdermales Applikationssystem mit einer Informationsschicht sowie ein Verfahren und eine Vorrichtung zur Herstellung eines solchen transdermalen Applikationssystems. Des Weiteren betrifft die vorliegende Erfindung die Verwendung eines solchen transdermalen Applikationssystems.

Transdermale Applikationssysteme, allgemein und im Folgenden auch als "TDS" (*englisch*: Transdermal Delivery System) bezeichnet, dienen dem Verabreichen von Arzneistoffen durch die Haut eines Patienten in dessen Blutkreislauf. Sie umfassen gewöhnlich ein Reservoir für den Wirkstoff, eine wirkstoffundurchlässige Rückschicht sowie eine Schutzfolie, die vor einer Applikation des Systems entfernt wird.

Transdermale Applikationssysteme enthalten den Wirkstoff oder die Wirkstoffformulierung in einer flächigen wirkstoffhaltigen Speicherschicht bzw. Speicherschichtanordnung, welche meist in Form einer Matrixschicht bzw. Matrixschichtanordnung ausgebildet ist. Eine solche flächige wirkstoffhaltige Matrixschicht kann z. B. als Klebstoffschicht ausgebildet sein, mit welcher diese an der Haut befestigt wird. Auch kann eine flächige wirkstoffhaltige Matrixschicht als feste, halbfeste oder flüssige Wirkstoffmatrix ausgebildet sein, welche keine adhäsiven Eigenschaften aufweist. In diesem Fall kann ein transdermales Applikationssystem mit einer zusätzlichen, wirkstoffhaltigen oder wirkstofffreien druckempfindlichen Kleberschicht versehen sein, mit welcher das TDS auf der Haut fixiert wird.

Bei transdermalen Applikationssystemen erfolgt der Wirkstoffeintrag in die Haut eines Patienten durch Diffusion des Wirkstoffs entlang des Wirkstoffkonzentrationsgefälles, das zwischen der wirkstoffhaltigen Speicherschichtanordnung bzw. wirkstoffhaltigen Matrix des TDS und dem vom Blutgefäßsystem durchsetzten Bereich der Haut besteht. Der mit einem transdermalen Applikationssystem erzielbare Wirkstoffblutspiegel wird, abgesehen von der Auflagefläche der Speicherschichtanordnung bzw. Matrix auf der Haut des Patienten, im Wesentlichen so über das Konzentrationsgefälle des Wirkstoffs an der Grenzschicht Haut zu Speicherschichtanordnung bzw. Matrix bestimmt.

An der der Haut abgewandten Seite ist die wirkstoffhaltige Speicherschichtanordnung, z. B. die wirkstoffhaltige Matrix, mit einer Rückschicht versehen, während auf der bei einem bestimmungsgemäßen Einsatz der Haut zugewandten Seite des TDS vor der Benutzung eine entfernbare Schutzfolie die Speicherschichtanordnung vor Verschmutzungen schützt, wodurch ein unkontrolliertes Austreten von Wirkstoff während einer Aufbewahrung des TDS verhindert wird.

Im Zuge der sicheren Verwendung von TDS werden diese im Allgemeinen auf ihrer äußeren Rückschicht mit einer Informationsschicht in Form einer Bedruckung versehen, welche beispielsweise sowohl einen Markennamen und/oder Wirkstoff als auch eine Dosis bzw. eine Abgaberate eines Wirkstoffs aus dem TDS deklariert. Eine gut haftende Bedruckung ist daher eine wesentliche Voraussetzung für eine sichere Verwendung bzw. Anwendung des TDS.

Jedoch ist die Oberfläche der Rückschicht des TDS sowohl während der Herstellung als auch während der Lagerung über Monate bis einige Jahre mechanischen Belastungen, insbesondere einem mechanischen Abrieb ausgesetzt.

TDS werden im Allgemeinen über einen Zeitraum von mindestens 24 Stunden, in einzelnen Fällen bis zu sieben Tagen auf der Haut des zu behandelnden Anwenders angebracht, wo sie bis zu deren Abnahme verbleiben. Häufig befindet sich eine Applikationsstelle für das TDS dabei am oberen Körper, wie beispielsweise im Bereich des Rückens, der Brust oder auch der Arme. An diesen Stellen ist die Rückschicht des TDS einem insbesondere durch Kleidung verursachten mechanischen Abrieb ausgesetzt, welcher einen Abrieb der Kennzeichnung eines TDS bis zu deren Unleserlichkeit verursachen kann. Auch kann eine Einwirkung von beispielsweise Duschwasser die Bedruckung bis zur Unkenntlichkeit beeinträchtigen.

Zudem ist die Auswahl von geeigneten Druckfarben begrenzt, da Druckfarben, welche toxische Inhaltsstoffe aufweisen können, nicht erwünscht sind, wenn diese mit einer wirkstoffhaltigen Matrix in Kontakt kommen könnten, da hierbei unerwünschte Wechselwirkungen des Wirkstoffs mit Bestandteilen der Druckerfarbe auftreten können, welche sich wiederum nachteilig auf die Behandlung bzw. Sicherheit des Anwenders auswirken können. Druckfarben, die für die Kennzeichnung von TDS verwendet werden dürfen, weisen jedoch in der Regel leider eine unzureichende Abriebfestigkeit auf.

Die Vergangenheit hat zudem gelehrt, dass Arzneimittel häufig gefälscht werden und den Patienten auf diese Weise auf illegalem Wege erreichen. Solche Arzneimittelfälschungen gefährden den Anwender in höchstem Maße und führen letztlich auch zu dessen Vertrauensverlust sowohl in das Produkt als auch in deren Lieferkette. Aus diesem Grunde werden zwischenzeitig von Seiten der medizinischen Zulassungsbehörden strenge Anforderungen an eine Fälschungssicherheit gestellt, wie beispielsweise aus der EU Direktive 2011/62/EU hervorgeht. Eine sichere Kennzeichnung des TDS ist also auch aus diesem Grunde von Bedeutung.

Die WO 99/12529 betrifft ein transdermales therapeutisches System, insbesondere ein wirkstoffhaltiges Pflaster, umfassend eine wiederablösbare Schutzschicht, eine haftklebende Reservoirschicht sowie eine - gegebenenfalls haftkleberbeschichtete - Rückschicht.

Die DE 19 708 674 A1 beschreibt ein transdermales therapeutisches System zur kontrollierten Abgabe von Wirkstoffen an menschliche oder tierische Haut, die sich durch eine geringe Applikationsdicke und hohe Flexibilität auszeichnen.Das Gebrauchsmuster G 94 09 784 U1 offenbart eine an der Hautoberfläche befestigbare Vorrichtung zur transdermalen Applikation eines Wirkstoffes mit einem schichtförmigen Trägerelement, das einen Aufnahmebereich für den Wirkstoff, eine Klebstoffschicht als Haftschicht, eine Abdeckschicht auf der der Haut abgewandten Seite, welche den Aufnahmebereich für den Wirkstoff überdeckt, wobei auf der der Haut abgewandten Seite des Trägerelements oder der Abdeckschicht Mittel zur Kennzeichnung vorgesehen sind.

Die WO 99/09960 A1 betrifft eine Kennzeichnung für klebende flächenförmige dermale und transdermale therapeutische Darreichungsformen unter Verwendung eines klebenden Pflasters als Informationsträger.

Die WO 87/02241 A1 beschreibt ein Verfahren zur Herstellung einer Darreichungs- und Dosierungsform für Arzneimittelwirkstoffe in Form einer Folie mit einer wirkstoffhaltigen Beschichtung, wobei a) aus einer wässrigen Zusammensetzung auf Basis von Stärken, Gelatinen, Glycerin und/oder Sorbit sowie gegebenenfalls natürlichen und/oder synthetischen Harzen und Gummen eine wasserlösliche Trägerfolie und b) eine wässrige Zusammensetzung aus dem Wirkstoff sowie Stärken, Gelatinen, Glycerin und/oder Sorbit sowie gegebenenfalls natürlichen und/oder synthetischen Harzen und Gummen hergestellt wird und die Beschichtungsmasse kontinuierlich mittels eines Walzenauftragverfahrens in genau vorbestimmter Menge auf mindestens eine Seite der Trägerfolie aufgebracht wird.

Schließlich offenbart die WO 00/04480 A1 eine Verfolgung und Identifizierung eines einzelnen Arzneimittelapplikationsgeräts, wie beispielsweise eines Pflasters, und beinhaltet die Kodierung des Arzneimittelapplikationsgeräts mit relevanten Informationen.

Es ist daher eine Aufgabe der vorliegenden Erfindung, ein transdermales Applikationssystem mit einer sichereren Informationsschicht bzw. Kennzeichnung sowie ein Verfahren zur Herstellung eines solchen TDS bereitzustellen. Auch ist es eine Aufgabe der vorliegenden Erfindung, ein entsprechendes TDS zur medizinischen, tiermedizinischen und kosmetischen Verwendung bereitzustellen.

Diese Aufgabe wird erfindungsgemäß durch ein TDS gemäß Anspruch 1 sowie durch ein Verfahren zur Herstellung eines solchen TDS gemäß Anspruch 12. Des Weiteren wird die Aufgabe durch ein erfindungsgemäßes TDS zur medizinischen, tiermedizinischen und kosmetischen Verwendung gemäß Anspruch 14 gelöst.

Ein erfindungsgemäßes transdermales Applikationssystem umfasst daher zumindest eine flächige wirkstoffhaltige Speicherschichtanordnung mit einer Applikationsseite und einer Rückseite. Die flächige wirkstoffhaltige Speicherschichtanordnung im Sinne der vorliegenden Erfindung kann in Form einer festen bis halbfesten Matrixanordnung oder in Form einer flüssigen Reservoiranordnung ausgebildet sein und bildet so das Wirkstoffdepot des transdermalen Applikationssystems. Auch kann ein erfindungsgemäßes transdermales Applikationssystem eine Matrixanordnung und eine Reservoiranordnung umfassen. Bevorzugt umfasst das erfindungsgemäße transdermale Applikationssystem jedoch eine Matrixanordnung.

Unter einer flächigen wirkstoffhaltigen Matrixanordnung oder Reservoiranordnung werden hierbei eine oder mehrere wirkstoffhaltige Matrix/ces bzw. Reservoir/e verstanden, welche horizontal in dem TDS, also waagrecht zur Oberfläche, angeordnet sind. Da eine Matrixanordnung in den meisten Fällen bevorzugt ist, wird die Speicherschichtanordnung im Folgenden auch oft als Matrixanordnung bezeichnet, wobei - sofern nicht anders erwähnt - andere Speicherschichtanordnungen nicht ausgeschlossen sein sollen.

Die im Allgemeinen zum Anbringen auf einer Haut vorgesehene bzw. mit der Haut in Kontakt kommende Seite des TDS wird als Applikationsseite bezeichnet. Die Applikationsseite kann hierfür ganzflächig haftklebend ausgebildet sein, beispielsweise indem die wirkstoffhaltige Speicherschichtanordnung bzw. die wirkstoffhaltige Matrixanordnung selbst haftklebend oder mit einem selbsthaftenden Kleber ganzflächig beschichtet ist oder nur teilflächig haftklebend ausgebildet ist.

Optional weist das TDS eine der Applikationsseite der wirkstoffhaltigen Speicherschichtanordnung zugewandte Schutzfolie auf.

Der Rückseite der Speicherschichtanordnung ist eine Rückschicht zugewandt. Erfindungsgemäß ist diese Rückschicht nun, wie unten erläutert wird, zumindest teilweise transparent und zwischen der Rückschicht und der Speicherschichtanordnung befindet sich zumindest eine Informationsschicht, wobei sich zwischen der Rückschicht und der Speicherschichtanordnung zumindest eine Zwischenschicht befindet und wobei die zumindest eine Zwischenschicht okklusiv ist. Eine solche Informationsschicht umfasst beispielsweise codierte und/oder lesbare Daten und/oder Zeichen, vorzugsweise optisch lesbare Daten und/oder Zeichen bzw. Zeichensätze oder -codes. Auch kann eine solche Informationsschicht ein Dekor und/oder ein Hologramm, also scheinbar dreidimensionale Informationen, bzw. Lentikularbilder o. Ä. umfassen.

Durch die Anordnung der Informationsschicht zwischen der Rückschicht und der Speicherschichtanordnung ist diese nun vor Abrieb und/oder Manipulationen erheblich besser geschützt und daher sicherer. Eine solche "abriebfeste Informationsschicht" bleibt nämlich vorteilhafterweise auch bei mechanischem Abrieb und/oder Belastung durch Lösungsvermittler wie Wasser (insbesondere Duschwasser) und/oder alkoholischen Lösungen oder ähnlichen Beanspruchungen der Rückseite des TDS möglichst ganzheitlich sichtbar. Dabei ist die Anbringung der Informationsschicht im Herstellungsprozess auch ohne oder mit nur geringem Mehraufwand möglich.

Gemäß einer bevorzugten Ausführungsform umfasst die Informationsschicht eine Bedruckung bzw. wird durch eine Bedruckung gebildet, insbesondere mit Druckfarben oder farbmittelhaltigen Gemischen unterschiedlicher Farbtöne (wie sie beispielsweise als HKS-Farbe von Hostmann-Steinberg Druckfarben, Kast + Ehinger Druckfarben und H. Schmincke & Co. erhältlich sind). Auch kann eine Farbwirkung einer Druckfarbe durch Mischungsverhältnisse der Vierfarbdruck-Farben Cyan, Magenta, Gelb und Schwarz (CMYK) erhalten werden. Solche Druckfarben oder farbmittelhaltigen Gemische können auch einen metallischen Glanz und/oder Interferenzerscheinungen aufweisen.

Das TDS weist dementsprechend bevorzugt einen zum Drucken geeigneten Bedruckstoff (DIN 16500, Teil 2) auf. Dabei können insbesondere die Rückschicht und/oder eine Zwischenschicht als Bedruckstoff ausgebildet sein, sofern diese beispielsweise mit Druckfarbe, Tinten oder Tonern und dergleichen bedruckt werden sollen.

Insofern das transdermale Applikationssystem auf der mit einer wirkstoffhaltigen Speicherschichtanordnung in Kontakt kommenden Seite bedruckt wird, berücksichtigt die Wahl der Bedruckung vorzugsweise Interaktionen der Druckfarbe mit der wirkstoffhaltigen Speicherschichtanordnung bzw. deren Inhaltsstoffen.

Wie oben erwähnt, weist das transdermale therapeutische System eine zumindest teilweise transparente Rückschicht auf. Eine solche zumindest teilweise transparente Rückschicht ist durchsichtig bis durchscheinend, jedoch nicht opak.

Eine Rückschicht im Sinne der vorliegenden Erfindung kann dabei aus einer oder mehreren Schichten, insbesondere auch Folienschichten, ausgebildet sein, wovon jedoch zumindest die Schicht bzw. Schichten, die zur Rückseite hin über der Informationsschicht liegen, zumindest teilweise transparent ausgebildet sind. Wenn eine Rückschicht zwei oder mehr Schichten, insbesondere Folien, umfasst, kann eine der Speicherschichtanordnung zugewandte innere Schicht sowohl auf der der Speicherschichtanordnung abgewandten Seite als auch - sofern die betreffende Schicht selber zumindest teilweise transparent ist - auf der der wirkstoffhaltigen Matrixanordnung zugewandten Seite eine Informationsschicht aufweisen.

Unter einer zumindest teilweise transparenten Rückschicht im Sinne der vorliegenden Erfindung wird eine Durchlässigkeit der Rückschicht in Bezug auf elektromagnetische Wellen, insbesondere von Licht, verstanden. Hierunter wird auch der Bereich zwischen sichtbarem Licht und längerwelliger Terahertzstrahlung, insbesondere von Infrarotstrahlung, verstanden.

Die Durchlässigkeit der zumindest teilweise transparenten Rückschicht in Bezug auf elektromagnetische Wellen, insbesondere von Licht, kann über die Transmission bzw. den Transmissionsgrad ermittelt werden und ist beispielsweise im Compendium of Chemical Terminology, IUPAC Recommendations ("Gold Book", A.D. McNaught and A. Wilkinson, 1997, Blackwell Science) beschrieben.

Eine zumindest teilweise transparente Rückschicht eines erfindungsgemäßen transdermalen Applikationssystems weist vorteilhaft eine Transmission von mindestens 5 %, bevorzugt von mindestens 10 %, besonders bevorzugt von mindestens 25 %, insbesondere von 50 %, insbesondere bevorzugt von mindestens 75 %, auf. Dabei kann die Rückschicht auch Bereiche aufweisen, welche nicht transparent sind.

Als Rückschichten eines erfindungsgemäßen TDS werden üblicherweise sogenannte Backingfolien aus beispielsweise Polyester mit einer Stärke von bis zu 500 µm, vorzugsweise aus dem Bereich von 5 bis 250 µm, besonders bevorzugt aus dem Bereich von 10 bis 100 µm, insbesondere aus dem Bereich von 20 bis 50 µm, insbesondere bevorzugt aus dem Bereich von 30 bis 40 µm, verwendet. Solche Backingfolien sind flexibel und können sich um die Ränder der Speicherschichtanordnung bzw. Matrix, d. h. um die in laterale Richtungen weisenden Seitenflächen der Speicherschichtanordnung legen und diese abdecken. Bevorzugt basiert eine Rückschicht auf einem Polymer ausgewählt aus der Gruppe bestehend aus Polyolefinen, Olefin-Copolymerisaten, Polyestern, Co-Polyestern, Polyamiden, CoPolyamiden, Polyurethanen und dergleichen. Als Beispiele für geeignete Materialien können Polyester und hiervon insbesondere Polyethylenterephthalate als auch Polycarbonate genannt werden, Polyolefine wie z. B. Polyethylene, Polypropylene oder Polybutylene, Polyethylenoxide, Polyurethane, Polystyrole, Polyamide, Polyimide, Polyvinylacetate, Polyvinylchloride, Polyvinylidenchloride, Co-Polymerisate, wie beispielsweise Acrylnitril-Butadien-Styrol-Terpolymere, oder Ethylen-Vinylacetat-Copolymerisate. Ein bevorzugtes Material einer Rückschicht ist ausgewählt aus einem Polyolefin und/oder einem Polyurethan, besonders bevorzugt einem Polyolefin und/oder Polyurethan-Schaummaterial.

Bei einer Ausführungsform des erfindungsgemäßen TDS kann wie oben erwähnt die Speicherschichtanordnung mit einer in der Fachsprache auch als Releaseliner bezeichneten abziehbaren Schutzfolie abgedeckt sein. Bei der Aufbewahrung des TDS wird hierdurch die Speicherschichtanordnung vor mechanischen Einflüssen und/oder einem unerwünschten Luftzutritt wirksam geschützt. Durch die Unterbindung eines unerwünschten Luftzutritts wird einer Zersetzung von in der Speicherschichtanordnung, insbesondere der Matrix, enthaltenen Wirkstoffen und insbesondere von sauerstoffempfindlichen Wirkstoffen vorgebeugt und so die Lager- bzw. Langzeitstabilität des Pflasters (nachfolgend wird das TDS auch als Pflaster bezeichnet) verbessert. Zur Applikation eines Pflasters wird dann vor der Befestigung des Systems auf der Haut zunächst die Schutzfolie des Pflasters abgezogen. Zum besseren Greifen und um dadurch ein Ablösen der Schutzfolie zu erleichtern, ragt die Schutzfolie bei einigen Pflastern über den Rand des restlichen Pflasters hinaus.

Bevorzugte Ausführungsformen eines transdermalen Applikationssystems umfassen wie erwähnt als Speicherschichtanordnung eine zumindest einen Wirkstoff enthaltende Wirkstoffmatrix und/oder -reservoir. Bestimmte Ausführungsformen können auch mehr als eine wirkstoffhaltige Matrix bzw. mehr als ein wirkstoffhaltiges Reservoir umfassen, wobei die verwendeten Wirkstoffe gleich oder unterschiedlich sein können und in unterschiedlicher Konzentration vorliegen können. So kann beispielsweise eine Speicherschicht ein Hormon aus der Gruppe der Östrogene enthalten und eine zweite oder weitere Speicherschicht ein Hormon aus der Gruppe der Gestagene. Selbstverständlich können auch Wirkstoffe unterschiedlicher Klassen, wie beispielsweise ein Antiemetikum in einem Wirkstoffdepot und ein Opioid in einem zweiten oder weiteren Wirkstoffdepot des erfindungsgemäßen TDS enthalten sein.

Schließlich müssen nicht alle Schichten des erfindungsgemäßen transdermalen Applikationssystems einen Wirkstoff enthalten, sondern das TDS kann zusätzlich zur zumindest einen wirkstoffhaltigen Speicherschicht, insbesondere einer wirkstoffhaltigen Matrixschicht, eine oder mehrere wirkstofffreie Schichten aufweisen.

Grundsätzlich ist es auch möglich, eine Speicherschichtanordnung hautseitig mit einer weiteren haftklebenden Schicht zu versehen.

Zur Ausbildung einer wirkstoffhaltigen Speicherschicht oder einer wirkstofffreien Schicht des transdermalen Applikationssystems eignen sich alle gewöhnlich für transdermale therapeutische Systeme geeigneten Materialien. Bevorzugt können einer Matrix z. B. Klebrigmacher zugesetzt werden, um zu einer selbstklebenden (d. h. haftklebenden) wirkstoffhaltigen Matrix zu gelangen, als Alternative oder zusätzlich zu der zuvor erwähnten hautseitigen haftklebenden Schicht. Auch kann die Matrix aus einem selbstklebenden Polymer aufgebaut sein. Hierbei zu nennen sind vor allem Polymere, die bei der Herstellung von transdermalen Systemen eingesetzt werden und physiologisch unbedenklich sind, wie z. B. Homo- und Copolymere von (Meth)acrylaten, Polyvinylether, Polyisobutylene, Polyisoprenkautschuke, Styrol-Butadien-Copolymere oder Styrol-Butadien-Styrol-Copolymere und Silicone. Von den (Meth)acrylat-Copolymeren können beispielsweise die Copolymere von Alkylacrylaten und/oder Alkylmethacrylaten und weiteren ungesättigten Monomeren genannt werden, wie Acrylsäure, Methacrylsäure, Acrylamid, Dimethylacrylamid, Dimethylaminoethylacrylamid, Acrylnitril und/oder Vinylacetat.

Die erfindungsgemäßen transdermalen Applikationssysteme sind zur Applikation von grundsätzlich allen Wirkstoffen oder Wirkstoffkombinationen geeignet. Unter einem Wirkstoff im Sinne der vorliegenden Erfindung werden dabei eine pharmazeutisch wirksame Substanz oder auch ein kosmetischer Wirkstoff und/oder Zusatzstoff und/oder Nährstoff oder Nahrungsergänzungsmittel und/oder ätherisches Öl verstanden.

Das erfindungsgemäße transdermale Applikationssystem gibt den Wirkstoff aus der flächigen Speicherschichtanordnung, bevorzugt der wirkstoffhaltigen Matrix, an die Haut ab, wobei zumindest ein Teil des Wirkstoffs systemisch aufgenommen werden kann. Das transdermale Applikationssystem kann daher auch zur dermalen Abgabe eines Wirkstoffs eingesetzt werden, wie beispielsweise zur Lokalanästhesie der Haut. Des Weiteren kann ein Applikationssystem im Sinne der vorliegenden Erfindung auch einen Duftstoff, wie beispielsweise ein ätherisches Öl aus einer Matrix, abgeben. Vorteilhaft kann der Duftstoff hierbei im Wesentlichen durch eine poröse Rückschicht in die Umgebung abgegeben werden.

Die Wirkstoffe können in verschiedenen Formen in der Zusammensetzung enthalten sein, je nachdem, welche Form die optimalen Abgabeeigenschaften des Wirkstoffs aus dem TDS ergibt. Im Falle von pharmazeutisch wirksamen Substanzen können diese in Form der freien Base oder Säure oder in Form von Salzen, Estern oder anderen pharmakologisch annehmbaren Derivaten oder als Komponenten von molekularen Komplexen vorliegen.

Die im Pflaster enthaltene absolute Wirkstoffmenge bestimmt im Allgemeinen die Zeitspanne, in der eine kontinuierliche Zufuhr in den Organismus aufrecht erhalten wird. Deshalb ist eine möglichst hohe Beladung der Speicheranordnung bzw. der Matrix mit Wirkstoffen dann wünschenswert, wenn die Applikationszeit eines Pflasters lang ist, d. h. mehrere Tage bis zu einer Woche beträgt.

Die vorliegende Erfindung betrifft somit auch die medizinische, tiermedizinische und/oder kosmetische Verwendung der erfindungsgemäßen Pflaster zur Abgabe von Wirkstoffen an und gegebenenfalls durch die Haut eines menschlichen oder tierischen Körpers und/oder an eine Umgebung um das Pflaster.

Des Weiteren betrifft die vorliegende Erfindung ein Verfahren zur Herstellung eines erfindungsgemäßen TDS, wobei das Verfahren folgende Schritte umfasst:
- Bereitstellen zumindest einer flächigen wirkstoffhaltigen Speicherschichtanordnung, vorzugsweise einer Matrixanordnung, mit einer Applikationsseite und einer Rückseite,
- optional Aufbringen einer Schutzfolie direkt oder indirekt auf die Applikationsseite der Speicherschichtanordnung,
- Aufbringen einer zumindest teilweise transparenten Rückschicht direkt oder indirekt auf die Rückseite der Speicherschichtanordnung,

wobei zwischen der Rückschicht und der Speicherschichtanordnung zumindest eine Informationsschicht eingebracht wird,
wobei zwischen der Rückschicht (5) und der Speicherschichtanordnung (1) zumindest eine Zwischenschicht (8) angeordnet wird, und
wobei die zumindest eine Zwischenschicht (8) okklusiv ist.

Unter Bereitstellen wird dabei sowohl eine Herstellung vor Ort als auch ein Zuliefern einer flächigen wirkstoffhaltigen Speicherschichtanordnung verstanden. Hierbei kann eine Speicherschichtanordnung, insbesondere flächige Matrixanordnung, bereits mit einer die Applikationsseite der Speicherschichtanordnung bedeckenden Schutzfolie versehen sein, welche im weiteren Verlauf der Herstellung auf dieser verbleibt oder optional in einem oder mehreren Herstellschritten durch eine alternative Schutzfolie ersetzt werden kann. Auch kann lediglich nur ein Teil einer Schutzfolie der bereitgestellten flächigen wirkstoffhaltigen Speicherschichtanordnung durch eine alternative Schutzfolie ersetzt werden.

Gemäß einer bevorzugten Ausführungsform können flächige Wirkstoff-Kerne, z. B. wirkstoffhaltige Matrixkerne, vor oder nach Aufbringen auf eine Schutzfolie aus der flächigen wirkstoffhaltigen Speicherschichtanordnung gestanzt und im weiteren Verlauf dem erfindungsgemäßen Herstellverfahren unterzogen werden. Insbesondere bevorzugt werden flächige Wirkstoff-Kerne aus einem Laminat, welches die flächige wirkstoffhaltige Speicherschichtanordnung und eine Schutzfolie umfasst, gestanzt.

In einem Herstellschritt wird wie erwähnt eine zumindest teilweise transparente Rückschicht direkt oder indirekt, also unmittelbar oder nach Einbringen von zumindest einer Zwischenschicht, in einem oder mehreren Herstellschritten auf die der Applikationsseite gegenüberliegende Rückseite der Speicherschichtanordnung bzw. einer Zwischenschicht aufgebracht. Bevorzugt wird eine solche zumindest teilweise transparente Rückschicht dabei auf eine Speicherschichtanordnung aufgebracht, welche bereits mit einer Schutzfolie versehen ist, jedoch kann eine solche transparente Rückschicht auch bereits vor Aufbringen einer Schutzfolie auf der der Applikationsseite der Speicherschichtanordnung gegenüberliegenden Seite aufgebracht werden.

Erfindungsgemäß wird wie erwähnt zumindest eine Informationsschicht zwischen die Rückschicht und die Speicherschichtanordnung eingebracht. Bevorzugt handelt es sich bei einer solchen Informationsschicht um eine Schicht, welche eine Bedruckung aufweist, insbesondere durch eine Bedruckung gebildet wird.

Eine Vorrichtung zur Herstellung der erfindungsgemäßen transdermalen therapeutischen Applikationssysteme kann folgende Komponenten aufweisen:
- eine erste Station zum Bereitstellen zumindest einer flächigen wirkstoffhaltigen Speicherschichtanordnung, vorzugsweise einer Matrixanordnung, mit einer Applikationsseite und einer Rückseite. Die Speicherschichtanordnung kann dabei in einem oder mehreren Schritten in der Station selbst hergestellt oder auch durch eine separate Vorrichtung, z. B. bei einem Zulieferer, hergestellt und in der Station zugeführt werden;
- optional eine zweite Station zum Aufbringen einer Schutzfolie direkt oder indirekt auf die Applikationsseite der Speicherschichtanordnung. Dies ist dann sinnvoll, wenn die (zum Beispiel zugelieferte) Speicherschichtanordnung nicht bereits eine Schutzfolie aufweist oder eine vorhandene Schutzfolie der Speicherschichtanordnung zumindest teilweise durch eine andere Schutzfolie ersetzt werden soll;
- eine dritte Station zum Aufbringen einer zumindest teilweise transparenten Rückschicht direkt oder indirekt auf die Rückseite der Speicherschichtanordnung;
- eine Applikationsstation, bevorzugt eine Druckerstation, welche so ausgebildet und geeignet angeordnet ist, um zwischen der Rückschicht und der Speicherschichtanordnung zumindest eine Informationsschicht einzubringen. Eine solche Applikationsstation ist vorzugsweise im Verhältnis zu den anderen Stationen so angeordnet, dass diese eine Informationsschicht wahlweise auf eine Rückschicht und/oder eine Zwischenschicht aufbringen kann, so dass sich eine Informationsschicht auf einer zur Rückschicht weisenden Zwischenschicht und/oder auf einer Innenseite, also auf einer zur Matrixanordnung gerichteten Seite der zumindest teilweise transparenten Rückschicht, befindet.

Weitere besonders vorteilhafte Ausgestaltungen und Weiterbildungen der Erfindung ergeben sich aus den abhängigen Ansprüchen sowie der vorhergehenden und nachfolgenden Beschreibung, wobei die Patentansprüche einer bestimmten Kategorie auch gemäß den abhängigen Ansprüchen einer anderen Kategorie weitergebildet sein können und Merkmale verschiedener Ausführungsbeispiele zu neuen Ausführungsbeispielen kombiniert werden können.

Gemäß einer Ausführungsform eines vorteilhaften transdermalen Applikationssystems ist eine Informationsschicht auf einer zur Speicherschichtanordnung gewandten Innenseite der Rückschicht aufgebracht, vorzugsweise aufgedruckt.

Ein TDS weist zwischen der Speicherschichtanordnung und der Rückschicht eine sogenannte Zwischenschicht auf. Eine solche Zwischenschicht kann dabei einen oder mehrere Haftkleber und eine oder mehrere Folien, sogenannte Zwischenfolien, aufweisen. Erfindungsgemäß ist eine Zwischenschicht okklusiv, d. h. als Sperrschicht für beispielsweise den Durchtritt von Wirkstoffen ausgebildet.

In einem entsprechenden bevorzugten Herstellverfahren wird zwischen die Speicherschichtanordnung und die Rückschicht eine Zwischenschicht eingebracht. Eine solche Zwischenschicht kann einen oder mehrere Haftkleber und eine Folie aufweisen.

Insbesondere basiert eine bevorzugte Zwischenschicht auf einem Polymer ausgewählt aus der Gruppe umfassend Polyolefine, Olefin-Copolymerisate, Polyester, Co-Polyester, Polyamide, Co-Polyamide, Polyurethane und dergleichen. Als Beispiele für geeignete Materialien können Polyester und hiervon insbesondere Polyethylenterephthalate als auch Polycarbonate genannt werden, Polyolefine wie z. B. Polyethylene, Polypropylene oder Polybutylene, Polyethylenoxide, Polyurethane, Polystyrole, Polyamide, Polyimide, Polyvinylacetate, Polyvinylchloride, Polyvinylidenchloride, Co-Polymerisate, wie beispielsweise Acrylnitril-Butadien-Styrol-Terpolymere, oder Ethylen-Vinylacetat-Copolymerisate.

Besonders bevorzugt ist eine Informationsschicht zwischen der Rückschicht und der Zwischenschicht eingebracht. Ganz besonders bevorzugt ist die Informationsschicht dabei eine Bedruckung, welche auf der der Rückschicht zugewandten Seite der Zwischenschicht aufgebracht wird. Alternativ oder zusätzlich kann eine Informationsschicht aber auch auf der der wirkstoffhaltigen Matrix zugewandten Seite der Rückschicht aufgebracht sein. In diesem Fall kann eine Informationsschicht, beispielsweise eine Bedruckung, bevorzugt spiegelverkehrt aufgebracht sein. Das Gleiche gilt, wenn die Informationsschicht auf einer der wirkstoffhaltigen Matrix zugewandten Seite einer Zwischenschicht aufgebracht wird, was insbesondere möglich ist, wenn die besagte Zwischenschicht auch zumindest teilweise transparent im Sinne der Erfindung ist.

Entsprechend wird nach einem vorteilhaften Herstellverfahren eine Informationsschicht zwischen die Rückschicht und die Zwischenschicht, bevorzugt auf die der Rückschicht zugewandten Seite, eingebracht.

Zum Schutz vor Arzneimittelfälschungen durch beispielsweise Eindringen von gefälschten bzw. manipulierten Arzneimitteln in die Lieferkette sowie auch im Zuge der Gewährleistung einer sicheren Anwendung über ein individuelles Erkennungsmerkmal weist eine Informationsschicht eines vorteilhaften transdermalen Applikationssystems ein oder mehrere Sicherheitsmerkmale auf. Ein solches Sicherheitsmerkmal ermöglicht es, die Echtheit des Arzneimittels zu überprüfen und zu identifizieren. Insbesondere ist ein Sicherheitsmerkmal dabei (nur) mit Hilfe von besonderen technischen Mitteln, insbesondere unter Bestrahlung mit Licht einer bestimmten Frequenz, beispielsweise in einer Laserlichtschranke, oder mit bestimmten Detektoren, auslesbar und enthält Angaben wie beispielsweise die Chargenbezeichung, das Verfallsdatum, die Produktnummer, Produktbezeichnung und Dosis oder auch eine weltweit eindeutige Kennung, anhand derer das erfindungsgemäße transdermale Applikationssystem identifizierbar ist. Ein bevorzugtes Sicherheitsmerkmal kann dabei ein Code, insbesondere ein 2D-Code, wie beispielsweise ein 2D-Matrix-Code und/oder ein Siegeldruck, sein. Auch bieten sich Informationsschichten in Form von Hologrammen bzw. Lentikularbildern besonders gut als Sicherheitsmerkmale an. Ebenso kann es zur Herstellung eines Sicherheitsmerkmals vorteilhaft sein, die Informationsschicht auf verschiedene Schichten zu verteilen, beispielsweise einen Teil der Informationen (spiegelverkehrt) auf die Innenseite der Rückschicht aufzubringen und einen anderen Teil auf eine der Rückschicht zugewandten Seite einer Zwischenschicht, so dass die Informationsteile nur bei einer korrekten Positionierung von Rückschicht und Zwischenschicht die gewünschte Information bilden. Dies erfordert einen besonders präzisen Produktionsaufwand, der eine Herstellung von Fälschungen erschwert und unter Umständen zu aufwändig macht.

Um eine optimale Haftung eines transdermalen Applikationssystems auf der Haut eines Anwenders zu gewährleisten, weist ein bevorzugtes TDS eine Rückschicht auf, welche eine Deckschicht bzw. ein Overtape umfasst bzw. bei dem die Rückschicht als Deckschicht bzw. Overtape ausgebildet ist.

Bei einem Overtape handelt es sich um ein mit einer wirkstofffreien Haftkleberschicht versehenes folienförmiges Material, das vorteilhaft wirkstoffundurchlässig ausgebildet oder mit einer wirkstoffundurchlässigen Rückschicht versehen ist. Das Overtape wird auf der der Haut abgewandten Seite des TDS über dem Laminat aus Speicherschichtanordnung, insbesondere wirkstoffhaltiger Matrix, und Rückschicht einer wirkstoffundurchlässigen Zwischenschicht angebracht und überragt dieses Laminat in lateraler Richtung, so dass der überstehende Klebstoffrand zur Befestigung auf der Haut dienen kann. Ebenso ermöglicht eine solche Deckschicht bzw. Overtape vorteilhaft ein in weiten Bereichen von den Klebeeigenschaften der Speicherschichtanordnung bzw. Matrix unabhängiges Anbringen des TDS auf der Haut einer Person oder eines Tieres. Bei sehr fließfähigen Matrices kann außerdem der kalte Fluss eingedämmt werden, wobei die vorhandene überstehende Rückschicht einen Übertritt von Wirkstoff aus der Matrix in die wirkstofffreie Kleberschicht der Deckschicht vorteilhaft verhindert.

Das Overtape kann vorzugsweise mehrschichtig ausgebildet sein. Insbesondere umfasst ein Overtape eine wirkstofffreie Kleberschicht und eine Overtape-Folie, wobei eine Overtape-Folie bevorzugt ein Polymer ausgewählt aus der Gruppe umfassend Polyolefine, Olefin-Copolymerisate, Polyester, Co-Polyester, Polyamide, Co-Polyamide, Polyurethane und dergleichen umfasst. Als Beispiele für geeignete Materialien können Polyester und hiervon insbesondere Polyethylenterephthalate als auch Polycarbonate, Polyolefine wie z. B. Polyethylene, Polypropylene oder Polybutylene, Polyethylenoxide, Polyurethane, Polystyrole, Polyamide, Polyimide, Polyvinylacetate, Polyvinylchloride, Polyvinylidenchloride, Co-Polymerisate, wie beispielsweise Acrylnitril-Butadien-Styrol-Terpolymere, oder Ethylen-Vinylacetat-Copolymerisate genannt werden. Ein bevorzugtes Material eines Overtapes ist ausgewählt aus einem Polyolefin und/oder einem Polyurethan, besonders bevorzugt einem Polyolefin und/oder Polyurethan-Schaummaterial.

Um eine Wanderung des Wirkstoffs aus der wirkstoffhaltigen Matrix durch eine Rückschicht und/oder ein Overtape zu verhindern, kann zumindest eine der wirkstoffhaltigen Matrix zugewandte Folie der Rückschicht okklusiv ausgebildet sein. Vorteilhaft weist ein transdermales Applikationssystem eine okklusive Rückschicht, insbesondere ein okklusives Overtape auf. Insbesondere umfasst ein Overtape der Rückschicht eine Schaumfolie.

Insofern ein transdermales Applikationssystem kein Overtape umfasst oder ein Overtape lediglich dem Schutz vor einem kalten Fluss der wirkstoffhaltigen Matrix dient, ist eine Speicherschichtanordnung, insbesondere eine flächige wirkstoffhaltige Matrixanordnung und/oder eine wirkstofffreie Matrixanordnung, eines bevorzugten TDS zumindest auf deren Applikationsseite haftklebend ausgebildet. Dies gewährleistet einen kontinuierlichen Kontakt der Applikationsseite mit der Haut und somit eine kontinuierliche Wirkstoffabgabe an bzw. durch die Haut eines Anwenders.

Die Menge des Wirkstoffs, der in der Zusammensetzung enthalten sein soll, variiert in Abhängigkeit von dem spezifischen Wirkstoff, der gewünschten therapeutischen Wirkung und der Zeitspanne, während der das TDS für eine Therapie sorgen soll. Bei den meisten Wirkstoffen ist der Durchtritt der Wirkstoffe durch die Haut der geschwindigkeitsbestimmende Schritt für deren Abgabe. Die Menge des Wirkstoffs und die Freisetzungsgeschwindigkeit werden also typischerweise so gewählt, dass man eine transdermale Abgabe erhält, die während einer längeren Zeitspanne durch eine Zeitabhängigkeit von im Wesentlichen nullter Ordnung gekennzeichnet ist.

Vorteilhaft kann die Menge des Wirkstoffs daher in dem System von etwa 0,3 Gew.-% bis etwa 50 Gew.-%, vorzugsweise von etwa 1,0 Gew.-% bis etwa 30 Gew.-%, besonders bevorzugt von etwa 2,5 Gew.-% bis etwa 20 Gew.-%, insbesondere von etwa 5 Gew.-% bis etwa 10 Gew.-%, variieren.

Beispielhafte aktive Arzneimittel bzw. Wirkstoffe, die in dem Pflaster der vorliegenden Erfindung enthalten sein können, sind:
1. Herzaktive Medikamente, zum Beispiel organische Nitrate, wie Nitroglycerin, Isosorbiddinitrat und Isosorbidmononitrat, Chinidinsulfat, Procainamid, Thiazide, wie Bendroflumethiazid, Chlorothiazid und Hydrochlorothiazid, Nifedipin, Nicardipin, adrenergische Blockiermittel, wie Timolol und Propranolol, Verapamil, Diltiazem, Captopril, Clonidin und Prazosin;
2. Androgene Steroide, wie Testosteron, Methyltestosteron und Fluoxymesteron;
3. Estrogene, wie konjugierte Estrogene, veresterte Estrogene, Estropipat, 17ß-Estradiol, 17ß-Estradiolvalerat, Equilin, Mestranol, Estron, Estriol, 17ß-Ethinylestradiol und Diethylstibestrol;
4. Gestagene, wie Progesteron, 19-Norprogesteron, Norethindron, Norethindronacetat, Chlormadinon, Ethisteron, Etonogestrel, Medroxyprogesteronacetat, Hydroxyprogesteroncaproat, Norethynodrel, Norelgestromin, 17α-Hydroxyprogesteron, Dydrogesteron, Dimethisteron, Ethinylestrenol, Norgestrel, Demegeston, Promegeston und Megestrolacetat;
5. Medikamente mit Wirkung auf das Zentralnervensystem, zum Beispiel Sedativa, Hypnotika, angstlösende Mittel, Analgetika und Anästhetika, wie Buprenorphin, Naloxon, Haloperidol, Fluphenazin, Pentobarbital, Phenabarbital, Secobarbital, Codein, Lidocain, Tetracain, Dibucain, Cocain, Procain, Mepivacain, Bupivacain, Etidocain, Prilocain, Benzocain, Fentanyl, Sufentanil, Alfentanil, Remifentanil, Tapentadol und Nicotin;
6. Nährstoffe und Nahrungsergängzungsmittel, wie Vitamine, essentielle Aminosäuren und essentielle Fette;
7. Entzündungshemmende Mittel, wie Hydrocortison, Cortison, Dexamethason, Fluocinolon, Triamcinolon, Prednisolon, Flurandrenolid, Methylprednisolon, Prednison, Methylprednisolon, Corticosteron, Paramethason, Betamethason, Ibuprofen, Naproxen, Fenoprofen, Fenbufen, Flurbiprofen, Ketoprofen, Suprofen, Indomethacin, Piroxicam, Aspirin, Salicylsäure, Diflunisal, Methylsalicylat, Phenylbutazon, Sulindac, Mefenaminsäure, Tolmetin und dergleichen;
8. Antihistaminika, wie Diphenhydramin, Dimenhydrinat, Perphenazin, Triprolidin, Pyrilamin, Chlorcyclizin, Promethazin, Carbinoxamin, Tripelennamin, Brompheniramin, Clorprenalin, Terfenadin und Chlorpheniramin;
9. Respiratorische Mittel, wie Theophillin und ß-adrenerge Agonisten, wie Albuterol, Terbutalin, Metaproterenol, Ritodrin, Carbuterol, Fenoterol, Chinterenol, Rimiterol, Solmefamol, Solerenal und Tetrochinol;
10. Sympathomimetika und Parasympathomimetika, wie Dopamin, Norepinephrin, Phenylpropanolamin, Phenylephrin, Physostigmin, Pseudoephedrin, Amphetamin, Propylhexedrin und Epinephrin;
11. Miotika, wie Pilocarpin und dergleichen;
12. Cholinergische Agonisten, wie Cholin, Acetylcholin, Methacholin, Carbachol, Bethanechol, Pilocarpin, Muskarin und Arecolin;
13. Antimuskarinische oder muskarinische cholinerge Gegenmittel, wie Atropin, Scopolamin, Methscopolamin, Homatropinmethylbromid, Methanthelin, Cyclopentolat, Tropicamid, Propanthelin, Dicyclomin und Eucatropin;
14. Mydriatika, wie Atropin, Cydoperitolat und Hydroxyamphetamin;
15. Psychoanaleptika, wie 3-(2-Aminopropyl)indol, 3-(2-Aminobutyl)indol und dergleichen;
16. Antiinfektionsmittel, wie Antibiotika, einschließlich Penicillin, Tetracyclin, Chloramphenicol, Sulfacetamid, Sulfamethazin, Sulfadiazin, Sulfamerazin, Sulfamethizol und Sulfisoxazol; antivirale Mittel; antibakterielle Mittel, wie Erythromycin und Clarithromycin, und andere Antiinfektionsmittel einschließlich Nitrofurazon und dergleichen;
17. Dermatologische Mittel, wie Vitamin A und Vitamin E;
18. Humorale Mittel, wie natürliche und synthetische Prostaglandine, zum Beispiel PGE1, PGE2a und PGF2a und das PGEr Analogon Misoprostol;
19. Antispasmodika, wie Atropin, Methanthelin, Papaverin und Methscapolamin;
20. Antidepressiva, wie Isocarboxazid, Phenelzin, Tranylcypromin, Imipramin, Amitriptylin, Trimipramin, Doxepin, Desipramin, Nortriptylin, Protriptylin, Amoxapin, Maprotilin und Trazodon;
21. Antidiabetika, wie Insulin, und Krebsmedikamente, wie Tamoxifen und Methotrexat;
22. Anorektika, wie Dextroamphetamin, Methamphetamin, Phenylpropanolamin, Fenfluramin, Diethylpropion, Mazindol und Phentermin;
23. Antiallergika, wie Antazolin, Methapyrilen, Chlorpheniramin, Mizolastin, Pyrilamin und Pheniramin;
24. Beruhigungsmittel, wie Reserpin, Chlorpromazin und angstlösende Benzodiazepine, wie Alprazolam, Chlordiazepoxid, Clorazepat, Halazepam, Oxazepam, Prazepam, Flurazepam, Triazolam, Lorazepam und Diazepam;
25. Antipsychotika, wie Thiopropazat, Chlorpromazin, Triflupromazin, Mesoridazin, Piperacetazin, Thiondazin, Acetophenazin, Fluphenazin, Perphenazin, Trifluoperazin, Chlorprathixen, Thiothixen, Haloperidol, Bromperidol, Loxapin und Molindon;
26. Abschwellende Mittel, wie Phenylephrin, Ephedrin, Naphazolin, Tetrahydrozolin;
27. Antipyretika, wie Aspirin, Salicylamid und dergleichen;
28. Antimigränemittel, wie Dihydroergotamin und Pizotylin;
29. Medikamente zur Behandlung von Übelkeit und Erbrechen, wie Chlorpromazin, Granisetron, Perphenazin, Prochlorperazin, Promethazin, Triethylperazin, Triflupromazin und Trimeprazin;
30. Antimalariamittel, wie 4-Aminochinolin, α-Aminochinolin, Chlorochin und Pyrimethamin;
31. Geschwürhemmende Mittel, wie Misoprostol, Omeprazol und Enprostil;
32. Peptide, wie wachstumshormonfreisetzender Faktor;
33. Medikamente für die Parkinson-Krankheit, Spastizität und akute Muskelspasmen, wie Rotigotin, Levodopa, Carbidopa, Amantadin, Apomorphin, Brorocripton, Selegilin (Deprenyl), Trihexyphenidylhydrochlorid, Benztropinmesylat, Procyclidinhydrochlorid, Baclofen, Diazepam und Dantrolen;
34. Antiestrogen- oder -hormonmittel, wie Tamoxifen oder humanes Chorion-Gonadotropin;
35. Aromatasehemmer, wie Anastrozol;
36. Cholinesteraseinhibitoren, wie Rivastigmin, Physostigmin, Galantamin oder Pyridostigmin;
37. Duftstoffe, wie ätherische Öle, wie Pfefferminzöl, Zitronenöl und dergleichen.

Eine kontinuierliche Abgabe eines Wirkstoffs an die Haut ist gerade für solche Krankheitsbilder wesentlich, für welche eine kontrollierte Therapie über Tage hinweg mit einer kontinuierlichen Abgabe des Wirkstoffs eine Grundvoraussetzung ist. Als bevorzugte Indikation ist hierbei die Therapie von Schmerzen, insbesondere von Krebsschmerzen und/oder neuropathischen und/oder Schmerzen des Muskel- und Bewegungsapparates zu nennen. Eine weitere bevorzugte Indikation ist die Hormonbehandlung, insbesondere die Empfängnisverhütung und/oder Hormonersatztherapie. Ebenfalls bevorzugt ist die Behandlung von altersbedingten Erkrankungen, insbesondere von Demenzformen, wie beispielsweise primäre und sekundäre Demenzformen und sonstigen neurodegenerativen Erkrankungen, wie Alzheimer-Krankheit, idiopathisches Parkinson-Syndrom und vaskuläre Demenz. Schließlich kann das erfindungsgemäße transdermale Applikationssystem insbesondere zur Verwendung bei der Behandlung von Bluthochdruck, Reisekrankheit, in der Palliativmedizin und zur unterstützenden Behandlung von Krebs, insbesondere von Brustkrebs, eingesetzt werden. Besonders bevorzugte transdermale Applikationssysteme enthalten daher einen Wirkstoff aus der Gruppe der Analgetika, insbesondere Buprenorphin und/oder Fentanyl und/oder Sufentanil und/oder Alfentanil und/oder Remifentanil und/oder Tapentadol, aus der Gruppe der Dopaminagonisten, insbesondere Rotigotin, aus der Gruppe der Cholinesteraseinhibitoren, insbesondere Rivastigmin, aus der Gruppe der Imidazoline, bevorzugt der alpha2-Adrenozeptor-Agonisten, insbesondere Clonidin, aus der Gruppe der Tropan-Alkaloide, bevorzugt der Agonisten der muskarinischen Acetylcholinrezeptoren, insbesondere Scopolamin, aus der Gruppe der Aromatasehemmer, insbesondere Anastrozol, oder aus der Gruppe der Hormone, insbesondere Estrogen und/oder Estradiol und/oder Ethinylestradiol, Norelgestromin und/oder Etonogestrel.

Weitere bevorzugte Wirkstoffe sind aus der Gruppe der ätherischen Öle ausgewählt, insbesondere aus Eukalyptusöl, Pfefferminzöl, Citronellöl, Kamillenöl, Campher, Menthol, Citrusöl, Zimtöl, Thymianöl, Lavendelöl, Nelkenöl, Teebaumöl, Cajeputöl, Niaouliöl, Kanukaöl, Manukaöl, Latschenkieferöl.

In einer vorteilhaften Ausführungsform enthält eine Speicherschichtanordnung, insbesondere in Form einer wirkstoffhaltigen flächigen Matrixanordnung, 0,1 bis 20 Gew.-%, besonders 0,5 bis 15 Gew.-%, insbesondere 1 bis 10 Gew.-% eines Wirkstoffs.

Ein erfindungsgemäßes TDS kann dabei auch als ein sogenanntes aktives transdermales Applikationssystem ausgebildet sein. Unter einem aktiven transdermalen Applikationssystem wird ein System verstanden, mit Hilfe dessen der Wirkstoff nicht oder nicht ausschließlich über Diffusion aus der Speicherschichtanordnung bzw. wirkstoffhaltigen Matrix abgegeben wird, sondern hierfür weitere technische Mittel, wie sie beispielsweise in iontophoretischen Systemen und/oder elektrophoretischen Systemen eingesetzt werden, wie beispielsweise eine Stromquelle, insbesondere eine Batterie, verwirklicht sind.

Auch kann eine Zusammensetzung des erfindungsgemäßen transdermalen Applikationssystems Mittel enthalten, die bekanntermaßen die Abgabe des Wirkstoffs durch die Haut beschleunigen. Diese Mittel werden als Hautpenetrationsverstärker, Beschleuniger oder Sorptionspromoter bezeichnet; sie werden hier kollektiv als "Verstärker" bezeichnet. Zu dieser Klasse von Mitteln gehören solche mit diversen Wirkungsmechanismen einschließlich solcher, die die Funktion haben, die Löslichkeit und das Diffusionsvermögen des Wirkstoffs in der Speicherschichtanordnung bzw. wirkstoffhaltigen Matrix zu verbessern, und solche, die die perkutane Resorption verbessern, indem sie zum Beispiel die Fähigkeit des Stratum comeum zur Feuchtigkeitsretention verändern, die Haut aufweichen, die Permeabilität der Haut verbessern, als Penetrationshilfsstoffe oder Haarfollikelöffner wirken oder den Zustand der Haut einschließlich der Grenzschicht verändern. Einige dieser Mittel können mehr als einen Wirkungsmechanismus aufweisen, aber letztlich dienen sie dazu, die Abgabe des Wirkstoffs aus dem erfindungsgemäßen Pflaster zu verbessern.

Einige Beispiele für Verstärker sind mehrwertige Alkohole, wie 4-Oxopentansäure, Dipropylenglycol, Propylenglycol und Polyethylenglycol, insbesondere 4-Oxopentansäure, die die Löslichkeit des Wirkstoffs erhöhen, Öle, wie Olivenöl, Squalen und Lanolin, Fettether, wie Cetylether und Oleylether, Fettsäureester, wie Isopropylmyristat, die das Diffusionsvermögen des Wirkstoffs erhöhen, Harnstoff und Harnstoffderivate, wie Allantoin, die die Fähigkeit von Keratin, Feuchtigkeit zurück zu halten, beeinflussen, polare Lösungsmittel, wie Dimethyldecylphosphoxid, Methyloctylsulfoxid, Dimethyllaurylamid, Dodecylpyrrolidon, Isosorbit, Dimethylacetonid, Dimethylsulfoxid, Decylmethylsulfoxid und Dimethylformamid, die die Permeabilität des Keratins beeinflussen, Salicylsäure, die das Keratin aufweicht, Aminosäuren, die Penetrationshilfsstoffe sind; Benzylnicotinat, das ein Haarfollikelöffner ist; aliphatische Tenside mit höherem Molekulargewicht, wie Laurylsulfatsalze, die den Oberflächenzustand der Haut und der verabreichten Wirkstoffe ändern. Zu den weiteren Mitteln gehören Olein- und Linoleinsäure, Ascorbinsäure, Panthenol, butyliertes Hydroxytoluol, Tocopherol, Tocopherylacetat, Tocopheryllinoleat, Propyloleat und Isopropylpalmitat.

Für Wirkstoffe, die eine geringe Löslichkeit in der wirkstoffhaltigen Matrix, bevorzugt in dem Polymersystem aufweisen, kann ein Co-Solvens für den Wirkstoff und das Polymer hinzugefügt werden. Co-Solventien, wie 4-Oxopentansäure, Lecithin, Retinolderivate, Tocopherol, Dipropylenglycol, Triacetin, Propylenglycol, gesättigte und ungesättigte Fettsäuren, Mineralöl, flüssiges Silikon, Alkohole, Butylbenzylphthalat und dergleichen, sind je nach der Löslichkeit des Wirkstoffs in dem TDS der vorliegenden Erfindung geeignet. Besonders geeignete Co-Solventien sind 4-Oxopentansäure und Tocopherol.

In bestimmten Ausführungsformen der Erfindung kann ein Weichmacher oder Klebrigmacher in der Zubereitung enthalten sein, um die Klebeeigenschaften der dermalen Zusammensetzung zu verbessern. Ein Klebrigmacher ist besonders nützlich bei denjenigen Ausführungsformen, bei denen der Wirkstoff das Polymer nicht weich macht. Geeignete Klebrigmacher sind dem Fachmann bekannt, einschließlich aliphatischer Kohlenwasserstoffe, gemischt aliphatischer und aromatischer Kohlenwasserstoffe, aromatischer Kohlenwasserstoffe, substituierter aromatischer Kohlenwasserstoffe, hydrierter Ester, Polyterpene und hydrierter Baumharze. Der eingesetzte Klebrigmacher ist vorzugsweise mit dem Gemisch der Polymere verträglich.

In bevorzugten Ausführungsformen handelt es sich bei dem Klebrigmacher um ein flüssiges Silikon (z. B. 360 Medical Fluid, erhältlich von der Dow Corning Corporation, Midland , M I) oder Mineralöl. Ein flüssiges Silikon ist geeignet für Gemische, die Polysiloxan als Hauptkomponente umfassen. In anderen Ausführungsformen, bei denen zum Beispiel ein Polyacrylat ein wesentlicher Bestandteil des TDS ist, ist 4-Oxopentansäure und/oder Mineralöl, insbesondere 4-Oxopentansäure, ein bevorzugter Klebrigmacher.

Die Zusammensetzung dieser Erfindung kann weiterhin mit verschiedenen Verdickungsmitteln, Füllstoffen und anderen Additiven, die zur Verwendung mit dermalen bzw. transdermalen Zusammensetzungen geeignet sind, versehen werden.

Die Konfiguration des transdermalen Abgabesystems der vorliegenden Erfindung kann jeder Form oder Größe entsprechen, die notwendig oder wünschenswert ist. Zum Beispiel kann eine vorteilhafte Dosierungseinheit eine Größe im Bereich von 1 bis 100 cm² aufweisen, bevorzugt im Bereich von 2,5 bis 60 cm², besonders bevorzugt im Bereich von 5 bis 40 cm², insbesondere im Bereich von 10 bis 25 cm².

Wie oben erläutert weist das transdermale Applikationssystem bevorzugt eine Schutzfolie auf. Bei einer bevorzugten Weiterbildung wird eine zweigeteilte Schutzfolie verwendet, deren aneinanderliegende Ränder beim Biegen des Pflasters aufstehen und so, ohne den wirkstoffhaltigen Bereich des Pflasters zu berühren, gegriffen werden können. Dabei kann eine Schutzfolie einen ersten Schutzfolienteil und einen zweiten Schutzfolienteil umfassen.

Ausführungsformen können zweckmäßig auch die Ausbildung der Schutzfolienteile aus unterschiedlichen Materialien umfassen, mit welchen unterschiedliche Haftungseigenschaften auf einer wirkstoffhaltigen Matrixanordnung erzielt werden können. Solche unterschiedlichen Haftungseigenschaften können wahlweise durch unterschiedliche Beschichtungen der Schutzfolie, wie zum Beispiel durch Silicon-, Fluorpolymer- oder Fluorsilicon-Beschichtung auf der der wirkstoffhaltigen Matrix zugewandten Seite erreicht werden. Besondere Ausführungsformen weisen einen ersten und einen zweiten Schutzfolienteil auf, die sich in ihren physikalischen und/oder optischen Eigenschaften und/oder ihrer Größe von dem zweiten Schutzfolienteil unterscheiden können.

Um die Gefahr der Kontamination der Finger beim Aufbringen des Pflasters auf die Haut gering zu halten, wird in der Regel empfohlen, zunächst nur einen Teil der zweigeteilten Schutzfolie abzuziehen, während das Pflaster gleichzeitig am vom anderen Teil der Schutzfolie bedeckten Bereich gehalten wird, den freigelegten Teil des Pflasters auf die Haut aufzubringen und anschließend den zweiten Teil der Schutzfolie abzuziehen. Um die Kontaminationsgefahr weiter zu reduzieren, sind die beiden Teile der Schutzfolie bei vorteilhaften Ausführungsformen überlappend angeordnet.

Die oben beschriebenen Ausführungsformen können bevorzugt gemäß einem Verfahren mit folgenden Schritten hergestellt werden:
(i) Bereitstellung eines wirkstoffhaltigen Laminats umfassend die Speicherschichtanordnung, insbesondere die flächige wirkstoffhaltige Matrixanordnung, eine Zwischenschicht und eine erste Prozessfolie;
(ii) Erzeugen, vorzugsweise Stanzen, von Wirkstoff-Kernen aus dem wirkstoffhaltigen Laminat;
(iii) Spenden der gestanzten Wirkstoff-Kerne aus Schritt (ii) auf eine Schutzfolie, wobei diese Schutzfolie auf der späteren Applikationsseite zum Liegen kommt;
(iv) Aufbringen der Rückschicht auf eine von der Schutzfolie abgewandten Seite der Wirkstoffkerne zum Erhalt eines Gesamt-Laminats;
(v) Vereinzeln der finalen TDS aus dem Gesamt Laminat, wobei jedes transdermale Applikationssystem zumindest einen Wirkstoff-Kern umfasst. Diese Vereinzelung kann beispielsweise in einem Stanz- und/oder Schneidevorgang erfolgen, wobei insbesondere die Rückschicht und die Schutzfolie durchtrennt werden;
(vi) Optional Verpacken der TDS in Beutel und vorzugsweise anschließendes Versiegeln.

Bei diesem bevorzugten Verfahren wird in einem Zwischenschritt, beispielsweise zwischen den Schritten (ii) und (iii) und/oder den Schritten (iii) und (iv), auf die Außenseite der Zwischenschicht der Wirkstoff-Kerne und/oder auf eine zu den Wirkstoff-Kernen weisende Innenseite der Rückschicht die Informationsschicht aufgebracht, vorzugsweise aufgedruckt.

Zur Einbringung der Informationsschicht zwischen Speicherschicht und Rückschicht kann in einem bevorzugten Verfahren vor oder nach Schritt (i) ein wirkstoffhaltiges Laminat umfassend eine Rückschicht und eine Prozessfolie auf einer zumindest teilweise transparenten Rückschicht auf der der wirkstoffhaltigen Matrix zugewandten und/oder abgewandten Seite bedruckt werden.

Ein weiteres bevorzugtes Verfahren kann an beliebiger Stelle vor Schritt (iv) die Herstellung eines wirkstofffreien Overtape-Laminats umfassend eine zumindest teilweise transparente Rückschicht und eine erste Prozessfolie vorsehen, wobei die zumindest eine teilweise transparente Rückschicht auf der der wirkstofffreien Matrix zugewandten Seite bedruckt sein kann. In einem solchen bevorzugten Verfahren kann ein Wirkstoff-Kern-Laminat nach oben genanntem Schritt (iii) oder (iv) mit dem Overtape-Laminat kaschiert werden, um so wiederum ein Gesamt-Laminat zu bilden.

Geeignete Materialien für die oben genannte Prozessfolie sind beispielsweise Polyester, Polypropylen, Polyvinylchlorid, Aluminium und Papier, wobei zumindest eine Seite der Prozessfolie eine Silicon-Beschichtung, Polyethylen-Beschichtung, Fluorsilicon-Beschichtung oder Polytetrafluorethylen-Beschichtung aufweist. Als Prozessfolie wird vorzugsweise eine Silicon-beschichtete Folie verwendet.

Schließlich umfasst die vorliegende Erfindung ein transdermales Applikationssystem, welches durch eines der Verfahren wie vorgehend beschrieben erhältlich ist.

Weitere Merkmale der Erfindung ergeben sich aus der nachfolgenden Beschreibung von Ausführungsbeispielen in Verbindung mit den Ansprüchen sowie den Figuren. Es sei darauf hingewiesen, dass die Erfindung nicht auf die Ausführungsformen der beschriebenen Ausführungsbeispiele beschränkt, sondern durch den Umfang der beiliegenden Patentansprüche bestimmt ist. Insbesondere können die einzelnen Merkmale bei erfindungsgemäßen Ausführungsformen in anderer Anzahl und Kombination als bei den untenstehend angeführten Beispielen verwirklicht sein. Bei der nachfolgenden Erläuterung einiger Ausführungsbeispiele der Erfindung wird auf die beiliegenden Figuren Bezug genommen. Es zeigen:
Figur 1 einen schematischen Längsschnitt durch ein erstes Ausführungsbeispiel eines erfindungsgemäßen TDS mit einer Informationsschicht, die auf einer Zwischenschicht aufgebracht ist,
Figur 2 eine schematische Draufsicht auf das Ausführungsbeispiel eines TDS gemäß Figur 1,
Figur 3 einen schematischen Längsschnitt durch ein zweites Ausführungsbeispiel eines erfindungsgemäßen TDS mit einer Informationsschicht, die auf einer Rückschicht aufgebracht ist,
Figur 4 ein Ablaufdiagramm für ein Ausführungsbeispiel eines erfindungsgemäßen Verfahrens zur Herstellung eines TDS,
Figur 5 eine schematische Darstellung eines Ausführungsbeispiels einer erfindungsgemäßen Vorrichtung zur Herstellung eines TDS gemäß dem Verfahren nach Figur 4.

In den Figuren sind Elemente, die im Wesentlichen gleiche technische Funktionen erfüllen, mit gleichen oder ähnlichen Bezugszeichen versehen.

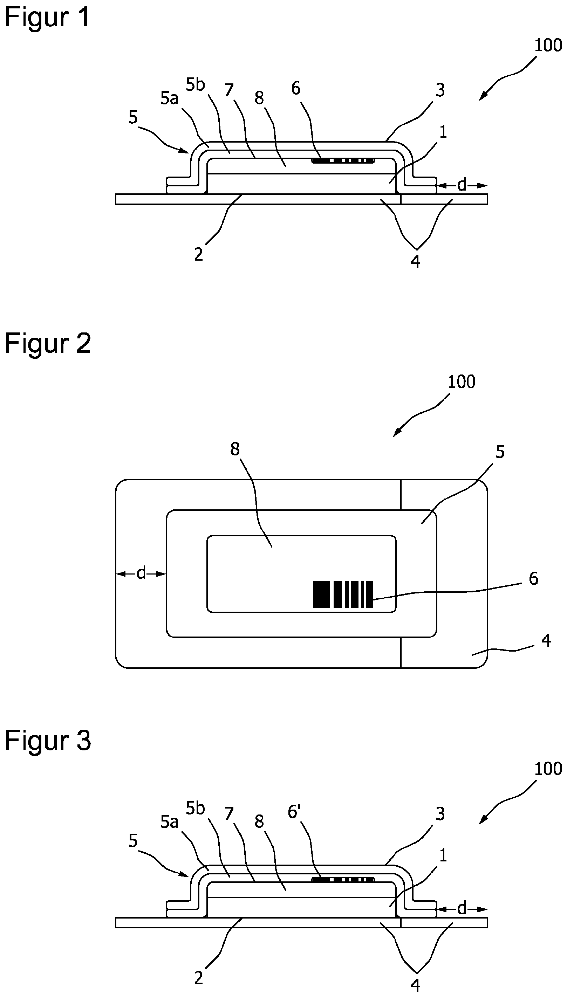

In den Figuren 1 und 2 wird schematisch eine erste Ausführungsform eines erfindungsgemäßen TDS 100 mit einer Informationsschicht gezeigt, welche zwischen der Speicherschichtanordnung und der Rückschicht angeordnet und daher vor Abrieb und anderen Beeinträchtigungen besser geschützt ist. Figur 1 zeigt dabei einen Längsschnitt durch das TDS 100 und Figur 2 eine Draufsicht auf die Rückseite des TDS 100.

Das TDS 100 weist als Speicherschichtanordnung 1 hier eine wirkstoffhaltige Matrix 1 auf, die auf der zum Kontakt mit der Haut vorgesehenen Seite, der Applikationsseite 2, von einer abziehbaren Schutzfolie 4 und auf der anderen Seite, der Rückseite 3, (indirekt) von einer wirkstoffundurchlässigen Rückschicht 5 abgedeckt ist, welche als Overtape 5 ausgebildet ist.

Um ein Entfernen der Schutzfolie 4 vor Applikation des TDS 100 von dessen Applikationsseite möglichst zu erleichtern, wird vorzugsweise eine beispielsweise Silicon-beschichtete Schutzfolie 4 verwendet. Geeignete Materialien für eine solche Schutzfolie 4 sind beispielsweise Polyester, Polypropylen, Polyvinylchlorid, Aluminium und Papier, wobei zumindest eine Seite der Schutzfolie 4 eine Silicon-Beschichtung, Polyethylen-Beschichtung, Fluorsilicon-Beschichtung oder Polytetrafluorethylen-Beschichtung aufweist.

Die zwischen Rückschicht 5 und Schutzfolie 4 angeordneten Seitenflächen der Matrix 1 begrenzen die Ausdehnung der Matrix 1 in der Fläche, d. h. in x- und y-Richtung (siehe Figur 2), und werden als Ränder der Matrix 1 bezeichnet. Die Rückschicht 5 bzw. das Overtape 5 überragt die Matrixränder in x- und y-Richtung, in der dargestellten Ausführungsform um eine Distanz d (siehe Figur 2). Dieser auch als Überlappung bezeichnete Überstand kann an allen Bereichen der Matrixränder konstant sein, kann an unterschiedlichen Bereichen der Matrixränder jedoch auch unterschiedliche Werte annehmen.

Die Matrix 1 eines TDS 100 weist üblicherweise eine Dicke im Bereich von 10 bis 500 µm auf, so dass durch den Überstand eines im vorliegenden Fall wirkstoffundurchlässigen Overtapes 5 über die Ränder der Matrix 1 ein wirksamer Schutz gegenüber einer ungewollten Kontaktierung der Matrixränder durch eine Person oder durch Verpackungsmaterialien gebildet wird. Die als Overtape 5 ausgebildete Rückschicht 5 dient zudem dem zuverlässigen großflächigen Anbringen des TDS 100 auf der Haut.

Im vorliegenden Fall besteht das Overtape 5 bzw. die Rückschicht 5 aus zwei Einzelschichten 5a, 5b. Die innere Schicht 5b dieser beiden Schichten 5a, 5b ist eine wirkstofffreie haftklebende Schicht 5b. Auf diese wirkstofffreie haftklebende Schicht 5b ist außen eine transparente Schaumfolie 5a aufgebracht.

Zwischen der Rückschicht 5 und der wirkstoffhaltigen Matrixanordnung 1 befindet sich eine Zwischenschicht 8, welche eine Informationsschicht 6 aufweist. Im vorliegenden Fall ist die Informationsschicht 6 als Druckschicht bzw. Aufdruck auf die Zwischenschicht 8, hier in Form eines aufgedruckten Barcodes, ausgebildet. Die Zwischenschicht 8 ist daher hier zumindest auf der zur Rückschicht 5 weisenden Seite als zum Drucken geeigneter Bedruckstoff (DIN 16500, Teil 2) ausgebildet. Da die Rückschicht 5 wie oben erläutert transparent ausgebildet ist, sind die Informationen der Informationsschicht durch die Rückschicht 5 hindurch gut erkennbar, wie dies in Figur 2 dargestellt ist.

In einer alternativen Ausführungsform, welche in Figur 3 dargestellt ist, ist die Informationsschicht 6' auf der Innenseite 7 der inneren Schicht 5a der Rückschicht 5 aufgebracht bzw. aufgedruckt. Dementsprechend ist diese Schicht 5a bedruckungsfähig bzw. als ein zum Drucken geeigneter Bedruckstoff ausgebildet. Die Informationen der Informationsschicht 6' sind hier spiegelverkehrt aufgebracht, so dass sie in der richtigen Orientierung durch die transparente Rückschicht hindurch lesbar bzw. maschinell auslesbar sind.

Die obigen Ausführungsbeispiele sind auch kombinierbar, d. h. dass sowohl auf der Rückschicht 5 als auch auf der Zwischenschicht 8 eine Informationsschicht aufgebracht ist. Ebenso wären mehrere Zwischenschichten denkbar, auf denen sich Informationsschichten befinden können. Zusätzlich oder alternativ kann sich auch eine Informationsschicht auf einer zur wirkstoffhaltigen Matrix gewandten Seite einer Zwischenschicht befinden. Selbstverständlich kann eine Rückschicht 5 auch zusätzlich auf ihrer außen liegenden Rückseite eine Bedruckung (wie bei herkömmlichen TDS) oder dergleichen aufweisen.

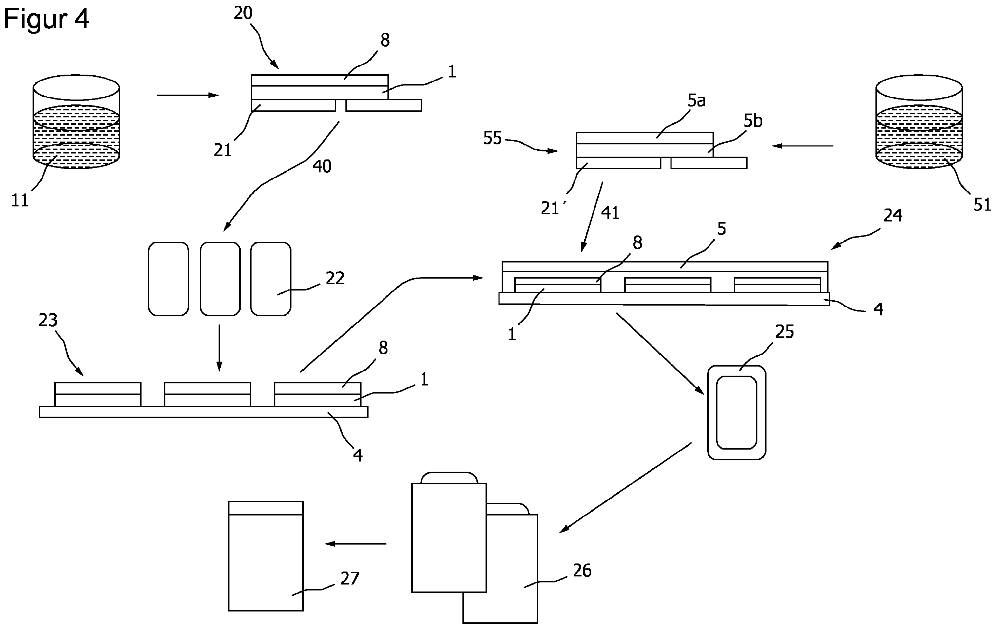

Figur 4 zeigt einen Ablauf für ein mögliches Verfahren zur Herstellung der erfindungsgemäßen transdermalen Applikationssysteme 100. Gemäß einem ersten bevorzugten Verfahren wird auf eine später die Zwischenschicht 8 bildende Folie zunächst eine flächige wirkstoffhaltige Matrix 1 aufgebracht. Dies erfolgt, indem ein wirkstoffhaltiges Matrixmaterial 11 auf eine Zwischenschicht 8 aufgebracht wird, beispielsweise mit Hilfe eines Rakels. Im vorliegenden Fall ist die Zwischenschicht 8 okklusiv ausgebildet. In einem nachfolgenden Schritt wird die Zwischenschicht 8 mit der wirkstoffhaltigen Matrix 1 auf der von der Zwischenschicht 8 weg weisenden Seite der Matrix 1 ganzflächig mit einer abziehbaren Prozessfolie 21 kaschiert, so dass die Prozessfolie 21 die flächige wirkstoffhaltige Matrix 1 bedeckt. Die Prozessfolie 21, die Matrix 1 und die Zwischenschicht 8 bilden so gemeinsam ein wirkstoffhaltiges Laminat 20. Als Prozessfolie 21 wird vorzugsweise eine Folie verwendet, die zumindest an der mit der wirkstoffhaltigen Matrix 1 in Kontakt tretenden Oberfläche siliconisiert ist und so ein späteres Abziehen der Prozessfolie 21 von der Matrix 1 erleichtert. Die Herstellung des wirkstoffhaltigen Laminats 20, d. h. der Verfahrensschritt I, kann dabei zu einem beliebigen Zeitpunkt vorher erfolgen. Beispielsweise kann das wirkstoffhaltige Laminat 20 auf einer Rolle oder dergleichen von einem anderen Betrieb bzw. Hersteller zugeliefert werden.

In einem weiteren Schritt II wird auf die Zwischenschicht 8 des wirkstoffhaltigen Laminats 20 die Informationsschicht 6 aufgebracht. Im vorliegenden Fall wird mit Hilfe einer Druckerstation 40 ein Sicherheitsmerkmal als Informationsschicht 6 aufgedruckt.

In einem nächsten Schritt III werden die Zwischenfolie 8 und die flächige wirkstoffhaltige Matrix 1 so gestanzt, dass sogenannte wirkstoffhaltige Matrix-Kerne 22 bzw. Wirkstoff-Kerne 22 entstehen.

Nach dem Abziehen (nicht in Figur 4 gezeigt) des nicht die Matrix-Kerne 22 bildenden Teils der Zwischenschicht 8 mit der die Matrix 1 bildenden Schicht (also des Stanzgitters) von der Prozessfolie 21 werden im Schritt IV die ausgestanzten Wirkstoff-Kerne 22, beispielsweise mithilfe eines Saughebers, von der Prozessfolie 21 entnommen und auf eine (finale) Schutzfolie 4 transferiert und bilden so - gemeinsam mit der Schutzfolie 4 - ein wirkstoffhaltiges Kern-Laminat 23. Als Schutzfolienmaterialien eignen sich beispielsweise Polyethylene, wie HDPE, Polypropylene, Polyester, Polysiloxane, Polyethylenterephthalate, Polyvinylchloride oder Polyurethane, gegebenenfalls in Form von Laminaten. Geeignete Schutzfolien sind beispielsweise von der Firma 3M unter der Markenbezeichnung Scotchpak^{®} (wie Scotchpak^{®} 1020, 1022, 9736, 9742, 9744, 9755), von der Firma Siliconature, z. B. unter der Bezeichnung Silthene oder Silphan, von der Firma Mitsubishi unter der Markenbezeichnung Hostaphan^{®} (z. B. GN, RD) oder von der Firma Loparex Inc. unter der Bezeichnung FL2000 erhältlich.

Es ist zu beachten, dass der Schritt 2, das Aufbringen bzw. Aufdrucken der Informationsschicht 6 auf die Zwischenschicht 8 des wirkstoffhaltigen Kern-Laminats 23, auch noch nach dem Schritt III oder dem Schritt IV durchgeführt werden kann, solange die Zwischenschicht 8 auf der von der Matrix 1 weg weisenden Seite noch nicht abgedeckt ist.

Schließlich wird im Schritt IV das wirkstoffhaltige Kern-Laminat 23 mit der wirkstofffreien Rückschicht 5, dem Overtape 5, kaschiert und bildet so ein Gesamt-Laminat 24.

Hierzu wird vorher in einem Schritt V ein Overtape-Laminat 55 erzeugt. Dabei wird auf eine Prozessfolie 21', welche gleich oder ähnlich wie die Prozessfolie 21 im Schritt I ausgebildet sein kann, zunächst eine wirkstofffreie haftklebende Matrix 51 als wirkstofffreie haftklebende Schicht 5b aufgetragen und anschließend mit einer Schaumfolie 5a kaschiert, welche so die äußerste Schicht des Overtape-Laminats 55 bildet. Diese Schaumfolie wurde zuvor mit üblichen Verfahren hergestellt oder kann als Rollenware käuflich erhalten werden.

Die Herstellung des wirkstoffhaltigen Overtape-Laminats 55, d. h. der Verfahrensschritt V, kann dabei auch zu einem beliebigen Zeitpunkt vorher erfolgen. Beispielsweise kann das Overtape-Laminat 55 auch auf einer Rolle oder dergleichen von einem anderen Betrieb bzw. Hersteller zugeliefert werden.

Die Prozessfolie 21' des Overtape-Laminats 55 wird vor dem Kaschieren des wirkstoffhaltigen Kern-Laminats 23 im Schritt VI wieder abgezogen.

Alternativ zur Aufbringung der Informationsschicht im Schritt II auf die Zwischenschicht 8 oder zusätzlich kann auch eine Informationsschicht 6' auf die Innenseite der Rückschicht 5 aufgebracht, bevorzugt aufgedruckt, werden. Dies könnte in einer Druckerstation 41 in dem optionalen Schritt V' erfolgen, nachdem die Prozessfolie 21' vom Overtape 5 abgezogen wurde, aber bevor das Overtape 5 auf das wirkstoffhaltige Kern-Laminat 23 aufkaschiert wird.

Im Gesamt-Laminat 24 deckt das Overtape 5 nach dem Kaschieren die Wirkstoff-Kerne 22 ab und legt sich zwischen diesen an die Oberfläche der Schutzfolie 4 an.

In einem nachfolgenden Schritt VII wird die Rückschicht 5 bzw. das Overtape 5 so gestanzt, dass Kerne von transdermalen Applikationssystemen mit überlappender Rückschicht 5 bzw. Overtape 5 auf der Schutzfolie 4 ausgebildet werden. Nach dem Entfernen des Overtape-Stanzgitters (nicht in Figur 4 gezeigt) werden schließlich die fertigen TDS 100 aus der Schutzfolie 4 durch Stanzen oder Schneiden vereinzelt.

Die einzelnen transdermalen Applikationssysteme 100 werden anschließend in Beutel verpackt 26 (Schritt VIII) und bei Bedarf versiegelt 27 (Schritt IX).

Bei einem zweiten bevorzugten Herstellungsverfahren wird das Matrixmaterial 11 zunächst auf eine Prozessfolie 21 aufgetragen, auf der das Matrixmaterial 11 zwar haftet, die aber von dieser im weiteren Verlauf des Verfahrens wieder einfach abgezogen werden kann. Als Prozessfolie 21 wird daher vorzugsweise eine beispielsweise Silicon-beschichtete Prozessfolie 21 verwendet.

Die wirkstoffhaltige flächige Matrix 1 wird in einem nachfolgenden Schritt mit einer Zwischenschicht 8 kaschiert, die eine stärkere Verbindung mit der Matrix eingeht als die Prozessfolie 21. In darauffolgenden Schritten wird wiederum der Verbund aus Zwischenfolie 8 und Matrix 1 zur Ausbildung der Wirkstoff-Kerne 22 gestanzt und das Stanzgitter, d. h. der nicht die Wirkstoff-Kerne 22 umfassende Teil des Verbunds aus Zwischenschicht 8 und Matrixbeschichtung 1 abgezogen. Das weitere Verfahren kann wie oben erläutert durchgeführt werden.

Außer den beschriebenen Schritten kann das Verfahren natürlich auch weitere Schritte aufweisen, die im Allgemeinen zur Herstellung eines bestimmten transdermalen Applikationssystems angewendet werden, wie beispielsweise einen Schritt zum Trocknen der aufgetragenen wirkstoffhaltigen Matrix 1 bzw. der wirkstofffreien Kleberschicht 5b o. ä. Zwischenschritte.

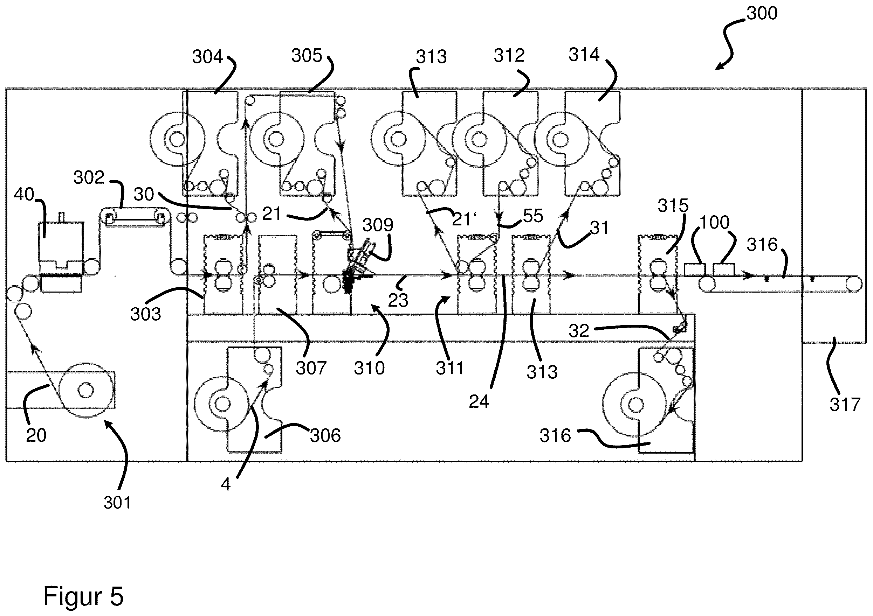

In Figur 5 ist eine Vorrichtung 300 in Form einer kompletten Anlage 300 dargestellt, mit der TDS 100 gemäß dem zuvor beschriebenen bevorzugten Verfahren hergestellt werden können. Diese Vorrichtung 300 bzw. Anlage 300 umfasst eine Vielzahl von Stationen.

In einer ersten Station 301 wird, wie dies im Zusammenhang mit Schritt I in Figur 4 erläutert wurde, ein wirkstoffhaltiges Laminat 20 zur Verfügung gestellt, welches eine flächige wirkstoffhaltige Speicherschichtanordnung, vorzugsweise die Matrix, umfasst, die auf ihrer Rückseite bereits mit einer Zwischenschicht belegt ist und auf ihrer späteren Applikationsseite auf einer Prozessfolie angeordnet ist. Dieses wirkstoffhaltige Laminat 20 wurde, wie oben erläutert, hergestellt und auf einer Rolle aufgewickelt, von der in der Station 301 dann das Laminat 20 abgewickelt und den nachfolgenden Stationen zugeführt wird. In diesem Zusammenhang wird darauf hingewiesen, dass in der Darstellung in Figur 5 neben den ausdrücklich bezeichneten Stationen eine Vielzahl von weiteren Rollen, insbesondere Umlenkrollen und/oder Spannrollen, etc. vorhanden sind, die dazu dienen, das von den Rollen kommende Bandmaterial, also beispielsweise die Laminate, Folien etc., und auch die zu entfernenden Restfolien, Stanzgitter etc. zu spannen und dafür zu sorgen, dass diese von den dafür vorgesehenen Rollen mit dem passenden Zug abgewickelt bzw. auf die jeweiligen Rollen wieder aufgewickelt werden, um so einen reibungslosen Prozessablauf zu gewährleisten.

In Figur 5 ist als nächste Station hinter der ersten Station 301, von der aus das wirkstoffhaltige Laminat 20 zugeführt wird, eine Applikationsstation 40, hier in Form einer Druckerstation 40, angeordnet, welche, wie dies im Zusammenhang mit Schritt II in Figur 4 erläutert wurde, die Zwischenschicht des Laminats 20 auf der Außenseite bedruckt. Diese Bedruckung erfolgt so, dass später in jedem einzelnen TDS eine entsprechende Bedruckung vorhanden ist, d. h. es wird hier dafür gesorgt, dass wiederkehrend entlang der Rolle und ggf. nach einem bestimmten Nutzenschema auch nebeneinander (quer zur Laufrichtung des Laminats 20) mehrere Nutzen aufgedruckt werden.

Über eine weitere Spannvorrichtung 302 gelangt das bedruckte Laminat 20 dann in eine Stanzstation 303, in der die Wirkstoff-Kerne aus dem Laminat ausgestanzt werden. Die Stanze der Stanzstation 303 durchstanzt dabei die Zwischenschicht und die die Matrix 1 bildende Schicht so, dass jeweils um die Aufdrucke, welche in der Druckstation 40 auf die Außenseite der Zwischenschicht aufgebracht wurden, einzelne Matrix-Kerne herausgestanzt werden. Das überschüssige Stanzgitter 30, d. h. die Bereiche der Zwischenschicht und der die Matrix 1 bildenden Schicht um die Matrix-Kerne herum, werden dann von einer Aufwickelstation 304 von der Prozessfolie 21 des Laminats 20, auf der dann nur die Matrix-Kerne verbleiben, abgezogen.

Der Restteil des Laminats 20 wird dann einer weiteren Station 310, der Transferstation 310, zugeführt, in der die einzelnen Matrix-Kerne auf eine Schutzfolie 4 übertragen werden, welche von einer Abwickelstation 306 kommt. Diese Schutzfolie 4 wird zuvor in einer Umlenkstation 307 umgelenkt und ebenfalls der Transferstation 310 zugeführt. Die Transferstation 310 weist ein sog. Spenderschwert 309 auf, welches die Matrix-Kerne 22 von der Prozessfolie 21 ablöst und auf die durch die Transferstation 310 laufende Schutzfolie 4 appliziert. Die nicht mehr benötigte Prozessfolie 21 wird auf einer Aufwickelstation 305 wieder auf eine Rolle aufgewickelt.

Die Schutzfolie 4 mit den in der Transferstation 310 aufgebrachten Matrix-Kernen bildet, wie oben beschrieben, das wirkstoffhaltige Kern-Laminat 23, welches dann einer Overtape-Station 311 zugeführt wird. Dieser Overtape-Station 311 wird von einer Abwickelstation 312 von einer Rolle ein fertiges Overtape-Laminat 55, bestehend aus einer Prozessfolie 21', einer darauf aufgebrachten haftklebenden Schicht und einer darauf wiederum aufgebrachten Schaumfolie zugeführt. Die Herstellung eines solchen Overtape-Laminats 55 wurde bereits im Zusammenhang mit Figur 4, Verfahrensschritt V, erläutert. In der Overtape-Station 311 wird die aus der haftklebenden Schicht und der Schaumschicht bestehende Rückschicht von der Prozessfolie 21' abgezogen und als Overtape rückseitig auf das Kern-Laminat 23 appliziert, so dass, wie oben im Zusammenhang mit Figur 4 beschrieben, ein Gesamt-Laminat 24 entsteht, mit der Schutzfolie 4, den darauf applizierten Wirkstoff-Kernen und dem darüber liegenden Overtape, welches zwischen den Wirkstoff-Kernen direkt an der Oberfläche der Schutzfolie 4 anliegt. Von der Overtape-Station 311 wird die nicht mehr benötigte Prozessfolie 21' zu einer Aufwickelstation 313 geführt und dort auf einer Rolle aufgewickelt.

Das Gesamt-Laminat 24 gelangt dann in der Prozessstrecke weiter an eine Overtape-Stanze 313, welche dafür sorgt, dass um die Wirkstoff-Kerne herum jeweils das Overtape durchstanzt wird. Die nicht mehr benötigten Teile des Overtapes, d. h. das Stanzgitter 31 des Overtapes, wird auf einer Aufwickelstation 314 wieder auf eine Rolle aufgewickelt.

Im weiteren Verlauf entlang der Prozessstrecke befinden sich also dann auf der Schutzfolie 4 jeweils die Wirkstoff-Kerne, welche jeweils von einem Overtape abgedeckt sind, wobei das Overtape allseits über die Wirkstoff-Kerne herüberragt. Da das Overtape zumindest teilweise transparent ist, scheint, wie dies im Zusammenhang mit Figur 2 dargestellt ist, der in der Druckstation 40 auf die Zwischenschicht aufgebrachte Aufdruck durch das Overtape hindurch.

Die Schutzfolie 4 mit den darauf befindlichen Wirkstoff-Kernen mit Overtape werden dann einer weiteren Stanzstation 315 zugeführt, welche die fertigen TDS aus der Schutzfolie 4 ausstanzt, wobei die Stanzung hier so erfolgt, dass die Schutzfolie 4 allseits in einem Abstand d (siehe Figur 2) vom Rand des Overtapes jedes Wirkstoff-Kerns durchstanzt wird. Dadurch entstehen die fertigen TDS 100, die auf einem Fließband 316 dann einer weiteren Verarbeitung in einer Verpackungsstation 317 zugeführt werden können. Das an der Stanzstation 315 entstehende Stanzgitter 32 der Schutzfolie 4 wird in einer Aufwickelstation 316 auf eine Rolle aufgewickelt.

Es wird abschließend noch einmal darauf hingewiesen, dass es sich bei den vorhergehend detailliert beschriebenen Beispielen lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können.

## Patentansprüche

1. Transdermales Applikationssystem (100) aufweisend
- zumindest eine flächige wirkstoffhaltige Speicherschichtanordnung (1), vorzugsweise eine Matrixanordnung (1), mit einer Applikationsseite (2) und einer Rückseite (3),
- optional eine der Applikationsseite (2) der Speicherschichtanordnung (1) zugewandte Schutzfolie (4),
- eine der Rückseite (3) der Speicherschichtanordnung (1) zugewandte Rückschicht (5),
wobei die Rückschicht (5) zumindest teilweise transparent ist und sich zwischen der Rückschicht (5) und der Speicherschichtanordnung (1) zumindest eine Informationsschicht (6, 6') befindet,
wobei sich zwischen der Rückschicht (5) und der Speicherschichtanordnung (1) zumindest eine Zwischenschicht (8) befindet,
wobei die zumindest eine Zwischenschicht (8) okklusiv ist.

2. Transdermales Applikationssystem gemäß Anspruch 1, wobei eine Informationsschicht (6) auf einer der Speicherschichtanordnung (1) zugewandten Innenseite (7) der Rückschicht (5) aufgebracht, vorzugsweise aufgedruckt, ist.

3. Transdermales Applikationssystem gemäß Anspruch 2, wobei eine Informationsschicht (6') auf der Zwischenschicht (8) aufgebracht, vorzugsweise aufgedruckt, ist.

4. Transdermales Applikationssystem gemäß einem der Ansprüche 2 bis 3, wobei Informationen der Informationsschicht (6, 6') auf der Innenseite der Rückschicht (5) und/oder auf einer der Speicherschichtanordnung (1) zugewandten Seite der Zwischenschicht (8) spiegelverkehrt angeordnet sind.

5. Transdermales Applikationssystem gemäß einem der vorstehenden Ansprüche, wobei die Informationsschicht (6, 6') ein Sicherheitsmerkmal umfasst.

6. Transdermales Applikationssystem gemäß einem der vorstehenden Ansprüche, wobei die Rückschicht (5) okklusiv ist.

7. Transdermales Applikationssystem gemäß einem der vorstehenden Ansprüche, wobei die Rückschicht (5) ein Overtape (5) umfasst.

8. Transdermales Applikationssystem gemäß einem der vorstehenden Ansprüche, wobei die Rückschicht (5) ein Schaummaterial (5a), insbesondere ein Polyolefin und/oder Polyurethan-Schaummaterial, umfasst.

9. Transdermales Applikationssystem gemäß einem der vorstehenden Ansprüche, wobei die Speicherschichtanordnung (1) und/oder eine flächige wirkstofffreie Schichtanordnung zumindest auf der Applikationsseite (2) haftklebend ausgebildet ist.

10. Transdermales Applikationssystem gemäß einem der vorstehenden Ansprüche, wobei die Speicherschichtanordnung (1), insbesondere die Matrixanordnung (1), einen Wirkstoff aus der Guppe der Analgetika, insbesondere Buprenorphin und/oder Fentanyl und/oder Sufentanil und/oder Alfentanil und/oder Remifentanil und/oder Tapentadol, aus der Gruppe der Dopaminagonisten, insbesondere aus der Gruppe der Cholinesteraseinhibitoren, insbesondere Rivastigmin, aus der Gruppe der Imidazoline, bevorzugt der alpha2-Adrenozeptor-Agonisten, insbesondere Clonidin, aus der Gruppe der Tropan-Alkaloide, bevorzugt der Agonisten der muskarinischen Acetylcholinrezeptoren, insbesondere Scopolamin, aus der Gruppe der Aromatasehemmer, insbesondere Anastrozol, oder aus der Gruppe der Hormone, insbesondere Estrogen und/oder Estradiol und/oder Ethinylestradiol, Norelgestromin und/oder Etonogestrel, umfasst.

11. Verfahren zur Herstellung eines transdermalen Applikationssystems gemäß einem der vorstehenden Ansprüche, wobei das Verfahren folgende Schritte umfasst:
- Bereitstellen zumindest einer flächigen wirkstoffhaltigen Speicherschichtanordnung (1), vorzugsweise einer Matrixanordnung (1), mit einer Applikationsseite (2) und einer Rückseite (3),
- optional Aufbringen einer Schutzfolie (4) direkt oder indirekt auf die Applikationsseite (2) der Speicherschichtanordnung (1),
- Aufbringen einer zumindest teilweise transparenten Rückschicht (5) direkt oder indirekt auf die Rückseite (3) der Speicherschichtanordnung (1),
wobei zwischen der Rückschicht (5) und der Speicherschichtanordnung (1) zumindest eine Informationsschicht (6, 6', 40, 41) eingebracht wird,
wobei zwischen der Rückschicht (5) und der Speicherschichtanordnung (1) zumindest eine Zwischenschicht (8) angeordnet wird, und
wobei die zumindest eine Zwischenschicht (8) okklusiv ist.

12. Verfahren nach Anspruch 11, mit folgenden Schritten:
(i) Bereitstellung eines wirkstoffhaltigen Laminats (20) umfassend die Speicherschichtanordnung (1), insbesondere Matrixanordnung (1), eine Zwischenschicht (8) und eine erste Prozessfolie (21),
(ii) Erzeugen, vorzugsweise Stanzen, von Wirkstoff-Kernen (22) aus dem wirkstoffhaltigen Laminat (20),
(iii) Spenden der gestanzten Wirkstoff-Kerne (22) auf eine Schutzfolie (4) zum Erhalt eines wirkstoffhaltigen Kernlaminats (23),
(iv) Aufbringen der Rückschicht (5) auf eine von der Schutzfolie (4) abgewandte Seite der Wirkstoff-Kerne (22) zum Erhalt des Gesamt-Laminats (24),
(v) Vereinzeln der transdermalen Applikationssysteme aus dem Gesamtlaminat, wobei jedes transdermale Applikationssystem zumindest einen Wirkstoff-Kern (22) umfasst,
(vi) optional Verpacken der TDS (25) in Beutel (26) und vorzugsweise anschließendes Versiegeln (27),
wobei auf eine Zwischenschicht (8), vorzugsweise auf eine Außenseite der Zwischenschicht (8), der Wirkstoff-Kerne (22) und/oder auf eine zu den Wirkstoff-Kernen (22) weisende Innenseite der Rückschicht (5) die Informationsschicht (6, 6') aufgebracht, vorzugsweise aufgedruckt (40, 41), wird.

13. Transdermales Applikationssystem (100) gemäß einem der Ansprüche 1 bis 12 zur Verwendung bei der medizinischen und tiermedizinischen Behandlung.

14. Verwendung eines transdermalen Applikationssystems (100) gemäß einem der Ansprüche 1 bis 12 zur kosmetischen Anwendung, wobei die in der Speicherschichtanordnung (1), insbesondere Matrixanordnung (1), enthaltenen Wirkstoffe bevorzugt ausgewählt sind aus einem kosmetischen Wirkstoff und/oder Zusatzstoff und/oder Nährstoff oder Nahrungsergänzungsmittel und/oder einem Duftstoff, wie beispielsweise einem ätherischen Öl, wie Pfefferminzöl, Zitronenöl und dergleichen.

## Claims

1. A transdermal delivery system (100) comprising
- at least one planar active ingredient-containing reservoir layer assembly (1), preferably a matrix assembly (1), with an application side (2) and a back side (3),
- optionally a protective film (4) facing the application side (2) of the reservoir layer assembly (1),
- a backing layer (5) facing the back side (3) of the reservoir layer assembly (1),
wherein the backing layer (5) is at least partially transparent and at least one information layer (6, 6') is located between the backing layer (5) and the reservoir layer assembly (1),
wherein at least one intermediate layer (8) is located between the backing layer (5) and the reservoir layer assembly (1),
wherein the at least one intermediate layer (8) is occlusive.

2. Transdermal application system according to claim 1, wherein an information layer (6) is applied, preferably printed, onto an inner surface (7) of the backing layer (5) facing the reservoir layer assembly (1).

3. Transdermal application system according to claim 2, wherein an information layer (6') is applied, preferably printed, onto the intermediate layer (8).

4. Transdermal application system according to any one of claims 2 to 3, wherein information of the information layer (6, 6') is arranged in a mirror-inverted manner on the inner side of the backing layer (5) and/or on a side of the intermediate layer (8) facing the reservoir layer assembly (1).

5. Transdermal application system according to any one of the preceding claims, wherein the information layer (6, 6') comprises a safety feature.

6. Transdermal application system according to any one of the preceding claims, wherein the backing layer (5) is occlusive.

7. Transdermal application system according to any one of the preceding claims, wherein the backing layer (5) comprises an overtape (5).

8. Transdermal application system according to any one of the preceding claims, wherein the backing layer (5) comprises a foam material (5a), in particular a polyolefin and/or polyurethane foam material.

9. Transdermal application system according to any one of the preceding claims, wherein the reservoir layer assembly (1) and/or a planar active ingredient-free layer assembly is designed to be adhesive at least on the application side (2).

10. Transdermal application system according to any one of the preceding claims, wherein the reservoir layer assembly (1), in particular the matrix assembly (1), comprises an active ingredient from the group of analgesics, in particular buprenorphine and/or fentanyl and/or sufentanil and/or alfentanil and/or remifentanil and/or tapentadol, from the group of dopamine agonists, in particular from the group of cholinesterase inhibitors, in particular rivastigmine, from the group of imidazolines, preferably alpha2-adrenoceptor agonists, in particular clonidine, from the group of tropane alkaloids, preferably agonists of the muscarinic acetylcholine receptors, in particular scopolamine, from the group of aromatase inhibitors, in particular anastrozole, or from the group of hormones, in particular estrogen and/or estradiol and/or ethinyl estradiol, norelgestromin and/or etonogestrel.

11. A method for the manufacture of a transdermal delivery system according to any one of the preceding claims, wherein the method comprises the following steps:
- providing at least one planar active-ingredient-containing reservoir layer assembly (1), preferably a matrix assembly (1), with an application side (2) and a back side (3),
- optionally applying a protective film (4) directly or indirectly to the application side (2) of the reservoir layer assembly (1),
- applying an at least partially transparent backing layer (5) directly or indirectly to the back side (3) of the reservoir layer assembly (1),
wherein at least one information layer (6, 6', 40, 41) is incorporated between the backing layer (5) and the reservoir layer assembly (1),
wherein at least one intermediate layer (8) is arranged between the backing layer (5) and the reservoir layer assembly (1), and
wherein the at least one intermediate layer (8) is occlusive.

12. A method according to claim 11, comprising the following steps:
(i) providing an active ingredient-containing laminate (20) comprising the reservoir layer assembly (1), in particular a matrix assembly (1), an intermediate layer (8) and a first process film (21),
(ii) forming, preferably punching, active ingredient cores (22) from the active ingredient-containing laminate (20),
(iii) placing the punched active ingredient cores (22) onto a protective film (4) to obtain an active ingredient-containing core laminate (23),
(iv) applying the backing layer (5) to a side of the active ingredient cores (22) facing away from the protective film (4) to obtain the complete laminate (24),
(v) separating the transdermal application systems from the complete laminate, wherein each transdermal application system comprises at least one active ingredient core (22),
(vi) optionally packaging the TDS (25) into pouches (26) and preferably subsequently sealing (27),
whereby the information layer (6, 6') is applied, preferably printed (40, 41), onto an intermediate layer (8), preferably onto an outer surface of the intermediate layer (8), of the active ingredient cores (22) and/or onto an inner side of the backing layer (5) facing the active ingredient cores (22).

13. A transdermal application system (100) according to any one of claims 1 to 12 for use in medical and veterinary therapy.

14. Use of a transdermal application system (100) according to any one of claims 1 to 12 for cosmetic use, wherein the active ingredients contained in the reservoir layer assembly (1), in particular the matrix assembly (1), are preferably selected from a cosmetic active ingredient and/or additive and/or nutrient or dietary supplement and/or a fragrance, such as an essential oil, for example peppermint oil, lemon oil and the like.

## Revendications

1. Système d'application transdermique (100), comportant
- au moins un élément plat à couche réservoir (1) contenant un principe actif, s'agissant préférentiellement d'un élément à matrice (1), pourvu d'une face d'application (2) et d'une face arrière (3),
- optionnellement, un film de protection (4) tourné vers la face d'application (2) de l'élément à couche réservoir (1),
- une couche arrière (5) tournée vers la face arrière (3) de l'élément à couche réservoir (1)
la couche arrière étant au moins partiellement transparente, et au moins une couche d'information (6, 6') se trouvant entre la couche arrière (5) et l'élément à couche réservoir (1), au moins une couche intermédiaire (8) se trouvant entre la couche arrière (5) et l'élément à couche réservoir (1),
l'au moins une couche intermédiaire (8) étant occlusive.

2. Système d'application transdermique selon la revendication 1, une couche d'information (6) étant mise en place, préférentiellement par impression, sur une face intérieure (7) de la couche arrière (5) qui est tournée vers l'élément à couche réservoir (1).

3. Système d'application transdermique selon la revendication 2, une couche d'information (6') étant mise en place, préférentiellement par impression, sur la couche intermédiaire (8).

4. Système d'application transdermique selon l'une des revendications 2 à 3, les informations de la couche d'information (6, 6') étant disposées en inversion spéculaire sur la face intérieure de la couche arrière (5) et/ou sur la face de la couche intermédiaire (8) qui est tournée vers l'élément à couche réservoir (1).

5. Système d'application transdermique selon l'une des revendications précédentes, la couche d'information (6, 6') comprenant une dispositif de sécurité.

6. Système d'application transdermique selon l'une des revendications précédentes, la couche arrière (5) étant occlusive.

7. Système d'application transdermique selon l'une des revendications précédentes, la couche arrière (5) comprenant un surbande (5).

8. Système d'application transdermique selon l'une des revendications précédentes, la couche arrière (5) comprenant un matériau de type mousse (5a), notamment un matériau de type mousse de polyoléfine et/ou polyuréthane.

9. Système d'application transdermique selon l'une des revendications précédentes, l'élément à couche réservoir (1) et/ou un élément plat à couche exempte de principe actif étant réalisé, au moins sur la face d'application (2), de manière à présenter une adhésif.

10. Système d'application transdermique selon l'une des revendications précédentes, l'élément à couche réservoir (1), plus particulièrement l'élément à matrice (1), comprenant un principe actif issu du groupe des analgésique, s'agissant notamment de buprénorphine et/ou fentanyl et/ou sufentanil et/ou alfentanil et/ou rémifentanil et/ou de tapentadol, issu du groupe des antagonistes de dopamine, plus particulièrement du groupe des inhibiteurs de cholinestérase, s'agissant notamment de rivastigmine, issu du groupe des imidazolines, plus particulièrement des agonistes du récepteur adrénergique alpha-2, s'agissant notamment de clonidine, issu du groupe de alcaloïdes tropaniques, préférentiellement des agonistes des récepteurs muscariniques de l'acétylcholine, s'agissant notamment de scopolamine, issu du groupe des inhibiteurs d'aromatase, s'agissant notamment d'anastrozole, ou issu du groupe des hormones, s'agissant notamment d'estrogène et/ou d'estradiol et/ou d'éthynylestradiol, norelgéstromine et/ou d'étonogestrel.

11. Procédé de fabrication d'un système d'application transdermique selon l'une des revendications précédentes, ledit procédé comprenant les étapes suivantes :
- fournir au moins un élément plat à couche réservoir (1) contenant un principe actif, préférentiellement un élément à matrice (1), pourvu d'une face d'application (2) et d'une face arrière (3),
- optionnellement mettre en place un film de protection (4) directement ou indirectement sur la face d'application (2) de l'élément à couche réservoir (1)
- mettre en place une couche arrière (5) au moins partiellement transparente, directement ou indirectement, sur la face arrière (3) de l'élément à couche réservoir (1),
au moins une couche d'information (6, 6', 40, 41) étant insérée entre la couche arrière (5) et l'élément à couche réservoir (1),
au moins une couche intermédiaire (8) étant disposée entre la couche arrière (5) et l'élément à couche réservoir (1), et
l'au moins une couche intermédiaire (8) étant occlusive.

12. Procédé selon la revendication 11, comportant les étapes suivantes :
(i) fournir un ensemble laminé (20) contenant un principe actif, lequel comprend l'élément à couche réservoir (1), plus particulièrement un élément à matrice (1), une couche intermédiaire (8) et un premier film technique (21),
(ii) fabriquer, de préférence par poinçonnage, des noyaux à principe actif (22) à partir de l'ensemble laminé (20),
(iii) déposer les noyaux à principe actif (22) poinçonnés sur un film de protection (4) pour obtenir un noyau laminé (23),
(iv) mettre en place la couche arrière (5) sur une face des noyaux à principe actif (22) qui n'est par tournée vers le film de protection (4), pour ainsi obtenir un ensemble laminé global (24),
(v) diviser ledit ensemble laminé global en systèmes d'application transdermiques séparés, chaque système d'application transdermique comprenant au moins un noyau à principe actif (22),
(vi) optionnellement, emballer les TDS (25) dans des sachets (26) puis, de préférence, procéder à un scellement (27),
la couche d'information (6, 6') étant mise en place, préférentiellement par impression (40, 41), sur une couche intermédiaire (8), de préférence sur une face extérieure de la couche intermédiaire (8), des noyaux à principe actif (22) et/ou sur une face intérieure de la couche arrière (5) qui est tournée vers les noyaux à principe actif (22).

13. Système d'application transdermique (100) selon l'une des revendications 1 à 12, destiné à l'utilisation dans un traitement médical et vétérinaire.

14. Utilisation d'un système d'application transdermique (100) selon l'une des revendications 1 à 12 pour un usage cosmétique, les principes actifs contenus dans l'élément à couche réservoir (1), s'agissant notamment d'une couche à matrice (1), étant préférentiellement choisis parmi un principe actif cosmétique et/ou un additif et/ou un nutriment ou complément alimentaire et/ou un parfum comme, par exemple, une huile essentielle telle que l'huile de menthe poivrée, l'huile de citron et des huiles apparentées.
